# EUROPEAN PATENT SPECIFICATION

(11) **EP 3 302 611 B1**
(45) Date of publication and mention of the grant of the patent: **12.01.2022**
(21) Application number: 16725649.4
(22) Date of filing: 29.04.2016
(51) Int. Cl.: A61M 1/16, B01J 20/02, B01J 20/06, B01J 20/34, B01J 20/28

(54) **ZIRCONIUM PHOSPHATE AND ZIRCONIUM OXIDE RECHARGER AND CONTROL LOGIC**
ZIRKONIUMPHOSPHAT- UND ZIRKONIUMOXIDLADER UND STEUERLOGIK
RECHARGEUR DE PHOSPHATE DE ZIRCONIUM ET D'OXYDE DE ZIRCONIUM ET LOGIQUE DE COMMANDE

(30) Priority: 26.05.2015 US 201514722068; 26.05.2015 US 201514722119
(43) Date of publication of application: 11.04.2018
(73) Proprietor: Medtronic, Inc., Minneapolis, Minnesota 55432 (US)
(72) Inventor: MENON, Kanjimpuredathil, Muralikrishna, Bangalore Karnataka 560037 (IN); MALAYATH, Sujeendran, Bangalore Karnataka 560100 (IN); GERBER, Martin, T., Maple Grove, MN 55369 (US); HOBOT, Christopher, M., Rogers, MN55374 (US); PATIL, Kaustubh, R., Bangalore Karnataka 560048 (IN); JEYACHANDRAN, Ramkumar, Bangalore Karnataka 560066 (IN); SAXENA, Sameer, Gwalior Madhya Pradesh 474009 (IN)
(74) Representative: Maschio, Antonio
(86) International application number: PCT/US2016/030320
(87) International publication number: WO 2016/191042

(56) References cited:
- WO-A1-2015/060914
- WO-A1-2015/199765
- WO-A1-2015/199863
- WO-A1-2015/199864
- US-A1- 2015 251 161
- US-A1- 2015 251 162

## Description

### FIELD OF THE INVENTION

The invention relates to control logic and processes for monitoring and controlling sorbent rechargers. The sorbent rechargers and related control logic and process algorithms monitor and test flow, temperature, conductivity, pressure, and volume, as well as the heaters and pumps, to ensure proper operation during recharging of zirconium phosphate, zirconium oxide, or both zirconium phosphate and zirconium oxide. In addition, the sorbent rechargers can perform periodic rinse cycles and check for appropriate chemical inputs for the recharging process using sensors as described herein.

### BACKGROUND

Zirconium phosphate and zirconium oxide are used in sorbent dialysis to remove waste and unwanted solutes from spent dialysate. Generally, zirconium phosphate removes ammonium, potassium, calcium, and magnesium ions from dialysate while the zirconium oxide removes anions such as phosphate or fluoride ions. Both materials are usually packaged together in a cartridge of some type or packed in separate cartridges. Usually, sorbent cartridges are discarded and replaced after use. The discarded sorbent cartridges are broken down and the individual materials separated from each other. Because zirconium phosphate and zirconium oxide are expensive and rechargeable, sorbent re-processers treat the recovered zirconium phosphate and zirconium oxide with chemical solutions. The recycling process requires transporting the materials to reprocessing facilities and involves laborious recycling steps in addition to recharging the sorbent materials. Further, the sorbent material cannot be immediately reused, and must be added to a new sorbent cartridge and repackaged for sale. Safe disposal of the chemical waste from solutions used to recharge the materials may also require additional steps such as neutralizing the recharging solutions. Conventional methods drive up costs and infrastructure requirements, and increase complexity and waste. Sorbent rechargers are described for instance in US2015/251161 A1 and US2015/251162 A1.

Hence, there is a need for systems and methods capable of ensuring that the recharging is properly carried out. The need extends to systems and methods for testing the fluid lines, communication systems, pumps, valves, and chemicals used in the recharging process. The need includes appropriate control logic and process algorithms for monitoring, testing, cycling, and operating sorbent rechargers.

### SUMMARY OF THE INVENTION

The first aspect of the invention is drawn to a sorbent recharger, comprising: a first receiving compartment for a first sorbent module; the receiving compartment having a first sorbent module inlet and a first sorbent module outlet; a first inlet line fluidly connected to the first sorbent module inlet; a first effluent line fluidly connected to the first sorbent module outlet; at least one of an disinfectant source, a brine source, a base source, and a water source fluidly connected to the first inlet line; at least a first pump positioned in the first inlet line for pumping fluid from the disinfectant source, brine source, and water source to the first sorbent module inlet; at least one flow sensor, at least one pressure sensor, at least one temperature sensor, and at least one conductivity sensor; and a control system in communication with at least one of the flow sensor, pressure sensor, temperature sensor or conductivity sensor; the control system for controlling the first pump, preferably said sorbent recharger further comprising a user interface in communication with the control system, wherein the control system determines whether at least one of the pressure, flow rate, temperature, and conductivity are within predetermined ranges, wherein the control system generates an alert indicating a leak when the pressure is below the predetermined range; and wherein the control system generates an alert indicating an occlusion when the pressure is above the predetermined range.

In any embodiment, at least one conductivity sensor is located upstream of the first sorbent module inlet; the control system can determine the flow rate and conductivity of a fluid upstream of the first sorbent module; the control system can generate an alert indicating a pump failure when the flow rate is below the predetermined range and the conductivity of a fluid upstream of the first sorbent module inlet is within a predetermined range; and the control system can generate an alert indicating a chemical run-out when the control system determines the flow rate is below the predetermined flow rate range and that the conductivity of the fluid upstream of the first sorbent module inlet is below the predetermined range.

In any embodiment, the recharger can have a heater and a heat exchanger in the first inlet line, the temperature sensor in communication with the control system; wherein the control system controls the heater based on data from the temperature sensor.

In any embodiment, the control system can generate an alert if the temperature in the first inlet line does not reach a predetermined temperature in a predetermined amount of time.

In any embodiment, the recharger can have a second temperature sensor in the first effluent line, wherein the control system generates an alert if the temperature in the first effluent line does not reach a predetermined temperature in a predetermined amount of time.

In any embodiment, the recharger can have a second receiving compartment for a second reusable sorbent module; the second receiving compartment comprising a second sorbent module inlet and a second sorbent module outlet; a second inlet line fluidly connected to the second sorbent module inlet; a second effluent line fluidly connected to the second sorbent module outlet; wherein the disinfectant source, the base source, and the water source are fluidly connected to the second inlet line; at least a second pump positioned in the second inlet line for pumping fluid from the disinfectant source, base source, and water source to the second sorbent module inlet; at least one flow sensor, at least one pressure sensor, at least one temperature sensor, and at least one conductivity sensor positioned in the second inlet line; wherein the control system is in communication with the second pump, and at least one of the flow sensor, pressure sensor, temperature sensor, and conductivity sensor positioned in the second inlet line.

In any embodiment, at least one conductivity sensor can be positioned in the first effluent line; wherein the control system controls the at least one pump to pump fluid from the disinfectant source, brine source, and/or water source through the first reusable sorbent module; and wherein the control system determines a conductivity of fluid in the first effluent line based on data from the conductivity sensor positioned in the first effluent line; wherein at least one conductivity sensor is positioned in the second effluent line; wherein the control system controls the second pump to pump fluid from the disinfectant source, base source, and/or water source through the second reusable sorbent module; and wherein the control system determines a conductivity of fluid in the second effluent line based on data from the conductivity sensor positioned in the second effluent line.

In any embodiment, the second effluent line can be fluidly connected to the first effluent line at a junction; and the recharger can have a static mixer at or downstream of the junction.

In any embodiment, the control system can calculate a neutralization ratio based on the conductivity of the fluid in the first effluent line and the conductivity of the fluid in the second effluent line; and wherein the control system controls the second pump and the first pump based on data from the conductivity sensor in the first effluent line and the conductivity sensor in the second effluent line; and the control system can control the first pump and second pump to generate a fluid with a pH within a predetermined pH range in the static mixer based on the neutralization ratio.

In any embodiment, the predetermined pH range can be between 5 and 9.

In any embodiment, the control system can control the first pump to convey fluid with an acidic pH through the first inlet line and control the second pump to convey fluid with a basic pH through the second inlet line concurrently.

In any embodiment, the control system can determine the flow rate, pressure, and conductivity of a fluid upstream of the first sorbent module at preset times.

In any embodiment, the control system can stop the second pump when the conductivity of the fluid in the second effluent line reaches a preset conductivity.

In any embodiment, control system can stop the first pump when the conductivity of the fluid in the first effluent line reaches a preset conductivity.

In any embodiment, wherein the control system can start the first pump and second pump when the conductivity in the first effluent line reaches a preset conductivity.

In any embodiment, the control system can calculate an amount of brine necessary for recharging a zirconium phosphate module based, at least in part, on the temperature in the first effluent line.

In any embodiment, the control system can control the first pump to pump water from the water source through the first inlet line after pumping a first fluid through the first inlet line and before pumping a second fluid through the first inlet line.

In any embodiment, the sorbent recharger can include a heat exchanger; the heat exchanger fluidly connected to the first inlet line and first effluent line.

In any embodiment, the control system can determine an amount of base pumped through the first inlet line.

Any of the features disclosed as being part of the first aspect of the invention can be included in the invention, either alone or in combination.

The second aspect of the invention is drawn to a method, comprising the steps of: pumping fluid from an disinfectant source, a base source, a brine source, a water source, or combinations thereof, through a recharging flow path to a first sorbent module; and determining a presence of a leak, occlusion, pump failure, or chemical run-out, wherein determining the presence of a leak comprises determining that a pressure in the recharging flow path is below a predetermined range, wherein determining the presence of an occlusion comprises determining that a pressure in the recharging flow path is above a predetermined range, wherein determining the presence of a pump failure comprises the steps of: determining that a flow rate in the recharging flow path is below a predetermined range; and determining that a conductivity at a sorbent module inlet of the first sorbent module is within a predetermined range, wherein determining the presence of a chemical run out comprises the steps of determining that a flow rate in the recharging flow path is below a predetermined range; and determining that a conductivity at a sorbent module inlet of the first sorbent module is below a predetermined range.

In any embodiment, the method can include the steps of pumping fluid from an disinfectant source, a base source, a brine source, a water source, or combinations thereof through the recharging flow path to a second sorbent module; and pumping fluid through a first effluent line fluidly connected to the first sorbent module and a second effluent line fluidly connected to the second sorbent module to a static mixer or a common reservoir.

In any embodiment, the method can include the steps of determining a conductivity of a fluid in the first effluent line and determining a conductivity of a fluid in the second effluent line; and calculating a neutralization ratio based on a conductivity of fluid in the first effluent line and the second effluent line; wherein the step of pumping fluid from the first effluent line and the second effluent line to the static mixer or common reservoir comprises controlling a flow rate of the fluid in the first effluent line and second effluent line based on the neutralization ratio to generate a fluid in the static mixer or common reservoir within a predetermined pH range.

In any embodiment, the first sorbent module can contain zirconium phosphate; and the fluid can contain a brine solution; and the method can include the steps of determining a temperature of the brine solution fluid in a first effluent line fluidly connected to the first sorbent module; and calculating an amount of brine necessary for recharging the zirconium phosphate based, at least in part, on the temperature in the first effluent line.

In any embodiment, the first sorbent module can contain zirconium oxide, and the method can include determining an amount of base pumped through the first sorbent module.

In any embodiment, the method can include pumping fluid from the disinfectant source into the first sorbent module; determining a conductivity in the first effluent line; and stopping pumping the fluid from the disinfectant source when the conductivity in the first effluent line is within a predetermined range.

In any embodiment, the method can include pumping water from the water source through into the first sorbent module at a predetermined time after stopping pumping fluid from the disinfectant source.

In any embodiment, the method can include venting the first sorbent module at a predetermined time after stopping pumping fluid from the disinfectant source.

In any embodiment, the method can include pumping fluid from the disinfectant source into the first sorbent module while venting the first sorbent module.

### BRIEF DESCRIPTION OF THE DRAWINGS

FIG. 1 illustrates operation process algorithms for conducting a recharger communication test.
FIG. 2 illustrates operational process algorithms for beginning disinfection of sorbent modules with a recharger.
FIG. 3 illustrates operation process algorithms for conducting a chemical and system test.
FIG. 4 illustrates control algorithms for disinfecting sorbent modules with a recharger.
FIG. 5 illustrates control algorithms for rinsing sorbent modules after disinfection.
FIG. 6 illustrates control algorithms for a first stage of the recharging process.
FIG. 7 illustrates control algorithms for a second stage of the recharging process.
FIG. 8 illustrates control algorithms for a first stage of a rinse process.

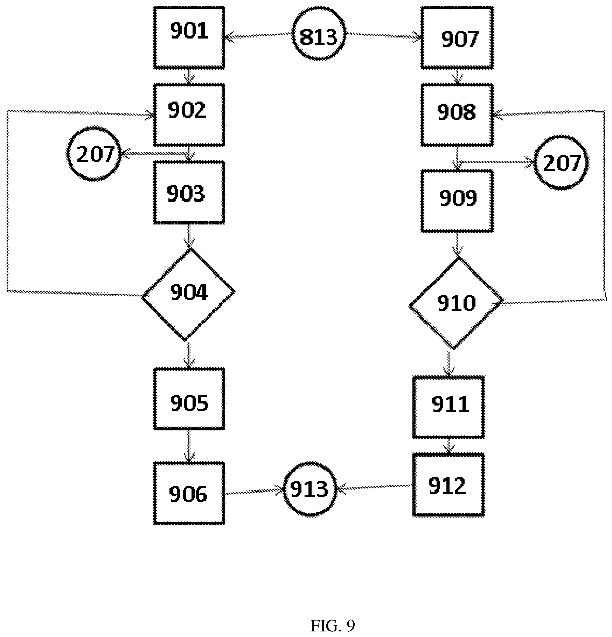

FIG. 9 illustrates control algorithms for a second stage of the rinse process.
FIG. 10 illustrates control algorithms for a third stage of the rinse process.
FIG. 11A shows a recharging flow path for recharging zirconium phosphate and zirconium oxide.
FIG. 11B shows a recharging flow path for recharging zirconium phosphate and is an exploded left side of FIG. 11A.
FIG. 11C shows a recharging flow path for recharging zirconium oxide and is an exploded right side of FIG. 11A.
FIG. 12A shows a recharging flow path for recharging zirconium phosphate and zirconium oxide with inline mixing of recharging solutions.
FIG. 12B shows a recharging flow path for recharging zirconium phosphate with inline mixing of recharging solutions and is an exploded right side of FIG. 12A.
FIG. 12C shows a recharging flow path for recharging zirconium oxide with inline mixing of recharging solutions and is an exploded left side of FIG. 12A.
FIG. 13 shows a timeline for concurrent recharging of zirconium oxide and zirconium phosphate.
FIG. 14 shows a recharger for recharging zirconium phosphate and zirconium oxide modules.
FIG. 15 shows a recharger fluidly connected to external fluid sources.
FIG. 16 shows material layers in a module sorbent cartridge including reusable modules.
FIG. 17 shows multiple sorbent modules connected together to form a sorbent cartridge.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used herein to refer to one or to more than one (*i.e.,* to at least one) of the grammatical object of the article. By way of example, "an element" means one element or more than one element.

The term "acidic pH" refers to an aqueous solution having a pH of less than 7.

An "alert" is any tactile, visual, or audio cue indicating the state of a system or component.

A "base source" is a fluid or concentrate source from which a basic solution can be obtained.

The term "basic pH" refers to an aqueous solution having a pH of greater than 7.

A "brine source" is a fluid or concentrate source from which a brine solution can be obtained. As used herein, a brine solution can refer to any solution comprising acids, bases and/or salts.

The terms "calculating a neutralization ratio" and to "calculate a neutralization ratio" refer to determining a relative amount of a first fluid necessary to neutralize a second fluid.

The terms "calculating an amount of brine necessary for recharging a sorbent module" or to "calculate an amount of brine necessary for recharging a sorbent module" refer to determining a volume of a brine solution that will result in recharging the sorbent module given a temperature, concentration, and flow rate of the brine solution.

The term "chemical mismatch" refers to a state in which an incorrect fluid is present in a fluid source.

The term "chemical run-out" refers to a state in which one or more chemicals are no longer available to a system.

A "common reservoir" can be a container for collecting fluid of any type from one or more fluid sources including fluid lines or other reservoirs. The "common reservoir" can for example, store used or waste fluids.

The term "communication" refers to an electronic link between two components.

The term "comprising" includes, but is not limited to, whatever follows the word "comprising." Use of the term indicates the listed elements are required or mandatory but that other elements are optional and may be present.

The term "concurrently" refers to two processes or events taking place at the same time.

The term "conductivity" refers to the inverse of the resistance of a material or fluid

A "conductivity sensor" is a sensor configured to measure the conductivity of a fluid.

The term "consisting of' includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The terms "contain," "containing," or "contained" as used herein means to keep a material within a specific place. "Contain" can refer to materials placed within a component, absorbed onto a component, bound to a component, or any other method of keeping the material in a specific place.

The term "control" or "controls" refers to the ability of one component to direct the actions of a second component.

A "control system" is any device which monitors and affects the operational conditions of a system. The operational conditions are typically referred to as output variables of the system wherein the output variables can be affected by adjusting certain input variables.

The terms "determining" and "determine" refer to ascertaining a particular state of a system or variable(s).

A "disinfectant source" is a fluid or concentrate source from which a disinfectant solution can be obtained. The disinfectant solution can be an acidic solution, such as a peracetic acid solution, or any other solution capable of disinfecting reusable sorbent modules.

The term "downstream" refers to a position of a first component in a flow path relative to a second component wherein fluid will pass by the second component prior to the first component during normal operation. The first component can be said to be "downstream" of the second component, while the second component is "upstream" of the first component.

An "effluent line" is a fluid passageway, tube, or path of any kind into which fluid exiting a container, module, or component will flow.

The term "flow rate" refers to a volume or quantity of liquid, gas, or a combination thereof, passing a particular point per unit time.

A "flow sensor" is a device capable of measuring an amount or rate of fluid, gas, or combination thereof, moving past or through a location.

A "fluid" is a liquid substance optionally having a combination of gas and liquid phases in the fluid. Notably, a liquid, as used herein, can therefore also have a mixture of gas and liquid phases of matter.

The term "fluidly connectable," "fluidly connect," "for fluid connection," and the like, refer to the ability of providing for the passage of fluid, gas, or combination thereof, from one point to another point. The two points can be within or between any one or more of compartments, modules, systems, components, and rechargers, all of any type. The connection can optionally be disconnected and then reconnected.

The terms "generates an alert" and to "generate an alert" refer to causing an alert to be created.

The terms "generates a fluid" and to "generate a fluid" refer to creating a fluid with a specified concentration, pH, temperature, and/or volume from one or more fluid sources.

A "heater" is a component capable of raising the temperature of a substance, container, or fluid.

The phrase "indicating a chemical-run out" refers to an alert that one or more chemicals are not available to the system.

The phrase "indicating a leak" refers to an alert that a leak potentially exists in the system.

The phrase "indicating an occlusion" refers to an alert that an occlusion potentially exists within a system.

The phrase "indicating a pump failure" refers to an alert that one or more pumps are not capable of pumping fluid at a desired flow rate.

An "inlet line" is a fluid line through which fluids can flow to enter a sorbent module.

A "junction" is a location where at least two fluid lines are connected to each other, with or without a valve.

The term "leak" refers to fluid exiting a fluid line or component at a location that the fluid is not intended to exit the fluid line or component.

The term "mixing" generally refers to causing one or more fluids from any source to combine together. For example, "mixing" can include turbulent flow at a location in a fluid line or a junction. Another example of "mixing" can include receiving one or more fluids in a component configured to receive fluids from one or multiple sources and to mix the fluids together in the component. Additionally, mixing can refer to the dissolution of a solid or solids with a fluid, wherein the solid or solids is dissolved in the fluid.

The term "neutralization ratio" refers to the relative amount of a first fluid necessary to neutralize a second fluid.

An "occlusion" is a blockage in a fluid line.

The term "positioned" or "position" refers to a physical location of a component or structure.

A "predetermined range" is a range of values for a variable that is calculated or determined prior to measuring the variable.

The term "preset" refers to particular time periods determined prior to a process.

The term "pressure sensor" refers to a device for measuring the pressure of a gas or liquid in a vessel, container, or fluid line.

The term "pump" refers to any device that causes the movement of fluids, gases, or combinations thereof, by applying suction or pressure.

The term "pump failure" refers to a state in which one or more pumps are not capable of pumping fluid at a desired flow rate.

The terms "pumping," "pumped," or to "pump" refer to moving a fluid, gas, or combination thereof, with a pump.

A "receiving compartment" is a space within a recharger into which a sorbent module to be recharged is placed.

A sorbent "recharger" is an apparatus designed to recharge at least one sorbent material.

"Recharging" refers to treating a sorbent material to restore the functional capacity of the sorbent material to put the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged." Recharging of rechargeable sorbent materials is not the same as replenishing of a sorbent material such as urease. Notably, urease is not "recharged," but can be replenished, as defined herein.

A "recharging flow path" is a path through which fluid can travel while recharging sorbent material in a reusable sorbent module.

The terms "sensing," "sensed" or to "sense" refer to determining one or more parameter or variable.

A "sensor" is a component capable of determining or sensing the states of one or more variables in a system.

A "sorbent cartridge module" or "sorbent module" means a discreet component of a sorbent cartridge. Multiple sorbent cartridge modules can be fitted together to form a sorbent cartridge of two, three, or more sorbent cartridge modules. In some embodiments, a single sorbent cartridge module can contain all of the necessary materials for dialysis. In such cases, the sorbent cartridge module can be a "sorbent cartridge."

A "sorbent module inlet" is a connector through which a fluid, slurry, or aqueous solution can enter a sorbent module.

A "sorbent module outlet" is a connector through which a fluid, slurry, or aqueous solution can exit a sorbent module.

The terms to "start a pump" or "starting a pump" refer to activating a pump to cause the pump to start pumping a fluid.

A "static mixer" is a component configured to receive fluids from one or multiple sources and to mix the fluids together. The static mixer may include components that agitate the fluids to further mixing.

The terms to "stop" a pump or "stopping a pump" refer to shutting off a pump to prevent the pump from pumping a fluid.

The term "temperature sensor" refers to a device for measuring the temperature of a gas or liquid in a vessel, container, or fluid line.

The term "upstream" refers to a position of a first component in a flow path relative to a second component wherein fluid will pass by the first component prior to the second component during normal operation. The first component can be said to be "upstream" of the second component, while the second component is "downstream" of the first component.

A "user interface" is a component that allows a user to communicate with a processor, computer, control system and the like. A user of the "user interface" can input information and can receive information from the processor or control system.

The term "venting" or "to vent" refers to opening one or more outlets in a container or module to allow fluid, gas, or a combination thereof, to escape.

A "water source" is a fluid source from which water can be obtained.

### Recharger Control Algorithms

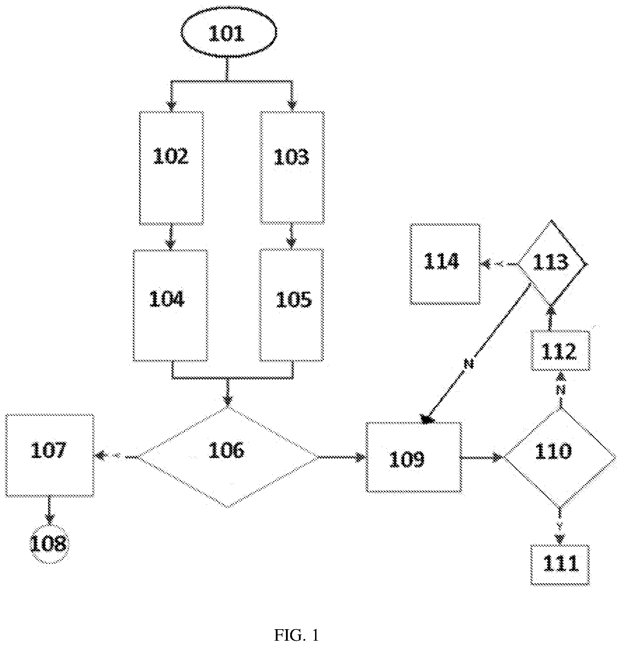

A recharger can be configured to recharge zirconium phosphate and zirconium oxide. To effectively and safely recharge the sorbent materials, operational process algorithms are needed in each step of the recharging process. FIG. 1 illustrates an operational process algorithm for ensuring proper communication between a recharger control system and zirconium phosphate and zirconium oxide recharging flow paths. The control system can be any component capable of monitoring and affecting the states of the recharger flow paths. The control system can use processors, memory and computer components to carry out the functions described. The control system is in communication with the pumps and valves of the recharger flow paths and can control the pumps and valves in accordance with stored instructions. The control system is also in communication with various sensors in the recharger flow paths. The control system receives data from the sensors and controls the pumps and valves of the recharger flow path on the basis of the data in accordance with stored instructions.

In step **101,** the user starts the recharging process. A recharger control system sends a test message to a zirconium phosphate control system in step **102** and to a zirconium oxide control system in step **103.** The zirconium phosphate control system sends a return message to the recharger control system in step **104,** and the zirconium oxide control system sends a return message to the recharger control system in step **105.** The zirconium phosphate and zirconium oxide control systems can also send test messages to each other. In step **106,** the recharger control system determines whether each of the return messages has been received. If so, in step **107,** the recharger control system sends messages to each of the zirconium phosphate and zirconium oxide control systems to begin the recharging process, indicated at step **108** in FIG. 1. If either of the return messages is not received by the recharger control system, the recharger control system generates an error message to the user indicating a communication failure. After the power up sequence is successful, the recharger control system communication link health is monitored by "Heartbeat Message." In step **109** a heartbeat message is sent to each of the zirconium phosphate and zirconium oxide control systems. The "heartbeat message" is a test message sent repeatedly at set intervals, such as once a second or once every 500 ms. One of skill in the art will understand that the interval between heartbeat messages can be set to any time. The recharger control system then checks to see if the heartbeat message was received in step **110.** If the heartbeat message has not been received, a counter is increased in step **112.** The recharger control system determines if the counter is above some pre-set threshold in step **113.** If the counter is above the pre-set threshold, an error message is generated in step **114,** indicating communication has been lost. If the counter is below the pre-set level, another heartbeat message is sent in step **109,** until the heartbeat message is received or the counter exceeds the pre-set threshold. The pre-set threshold of the counter can be any number, including between 1 and 5 or greater. If the heartbeat message is received, the counter is reset in step **111** and the heartbeat message is sent again at set intervals.

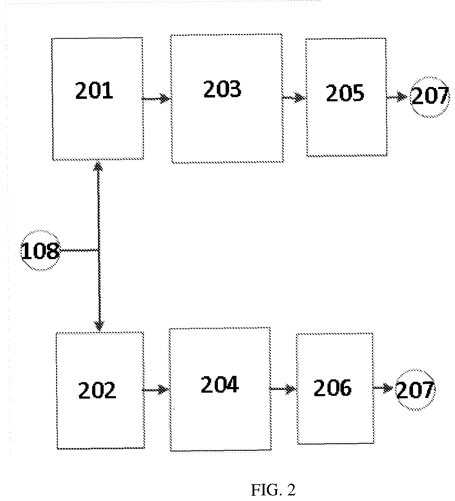

Once communication has been established between the recharger control system and each of the zirconium oxide, the recharging process can begin, as illustrated in FIG. 2, starting with step **108** of FIG. 1. The control system controls the pumps, valves, heaters and other components to generate and deliver the recharging fluids with the correct concentrations, flow rates, and temperature. The control system can communicate with the components of the recharging flow paths through any means known in the art, including WiFi, Bluetooth, or any other method. The control system sends instructions to the pumps, valves, heaters, or other components that cause these components to carry out the functions described. The recharger control system sends a message to the zirconium phosphate control system in step **201** and to the zirconium oxide control system in step **202** to begin a disinfection process. In step **203,** the zirconium phosphate control system activates one or more pumps and valves within a zirconium phosphate recharging flow loop to begin filling the zirconium phosphate module with a disinfection solution from a disinfectant source. The disinfectant can be any disinfectant capable of disinfecting the sorbent modules and compatible with zirconium phosphate and zirconium oxide, including peracetic acid, bleach, or citric acid. In step **204,** the zirconium oxide control system activates a different set of pumps and valves to begin filling the zirconium oxide control system with the disinfection solution. The disinfection solution is passed through the zirconium phosphate recharging flow path in step **205** and through the zirconium oxide recharging flow path in step **206.** Each of the zirconium phosphate and zirconium oxide control systems can begin a chemical and system test procedure in step **207.**

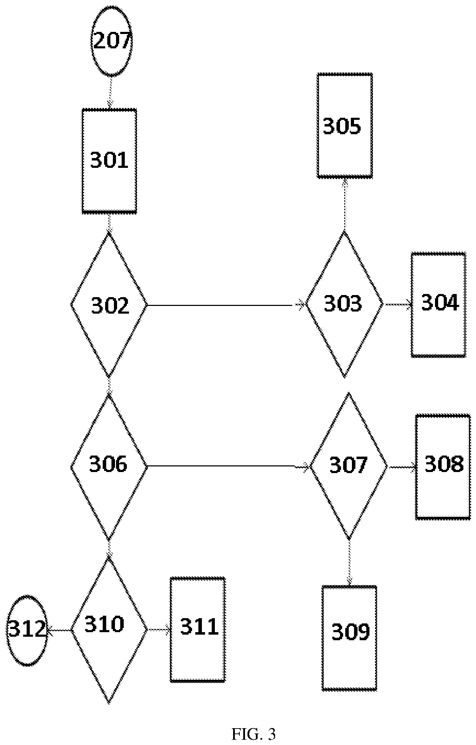

As illustrated in FIG. 3, the chemical and system test procedure begins in step **207.** In step **301,** the system monitors the pressure in the fluid lines with one or more pressure sensors in communication with the control system. In step **302,** the system determines whether the pressure as sensed by the pressure sensor is within a predetermined range. If the pressure is outside of the predetermined range, the system determines whether the pressure is above or below the predetermined range in step **303.** If the pressure is below the predetermined range, the system generates an alert indicating a leak or rupture of a fluid line in step **304.** If the pressure is above the predetermined range, the system generates an alert indicating an occlusion of the fluid lines in step **305.** The predetermined range can be any range of pressures high enough to move fluid throughout the recharging flow paths but low enough to avoid damage to the lines and components. The lower range depends on the flow rate and is adjustable. The higher range is generally 2.0 bar, but can depend on the capabilities of the system. In any embodiment, the predetermined range can be between 1.0 and 2.0 Bar.

If the pressure is within the predetermined range, the system determines whether the flow rates of the fluid moving through the recharging flow paths are within a predetermined range with one or more flow sensors in communication with the control system in step **306.** The predetermined range of flow rates can vary with the recharging process, as explained. In any embodiment, the predetermined range of the flow rates can be ± 10% of the set flow rate. Alternatively, the predetermined range can be a flow rate deviation, such as ± 20 mL/min of the set flow rate. If the sensed flow rates are outside of, or below, the predetermined range, the system determines the conductivity of the fluid at or upstream of the sorbent module inlets with a conductivity sensor in step **307.** If the conductivity of the fluid is determined to be 0, or below a predetermined low range, the system generates an alert indicating a chemical run-out in step **308.** If the conductivity of the fluid is greater than 0, or within a predetermined range, the system generates an error message indicating a pump failure in step **309.**

If the fluid flow rates are determined to be within the predetermined range in step **306,** the system determines whether the conductivity of the fluid at or upstream of the sorbent module inlets is within a predetermined range of an expected conductivity in step **310.** The expected conductivity will vary depending on the particular solution being pumped through the recharging flow paths during the recharging process. In any embodiment, the predetermined range can be ± 10% of the expected conductivity. If the conductivity is outside of the predetermined range, the system generates an alert indicating a possible chemical mismatch in step **311.** If the conductivity is within the predetermined range, the system sends a message to the zirconium phosphate and zirconium oxide control systems to continue the recharging process in step **312.** One of skill in the art will understand that the order of operations in chemical and system test procedure can be varied. For example, the system can determine whether the fluid flow rates are within a predetermined range prior to determining whether the pressure is within a predetermined range. Although shown as occurring at discrete times in the control algorithms, one of skill in the art will understand that the chemical and system test illustrated in FIG. 3 can be conducted at any time during the recharging process, including at preset times.

To avoid occlusion of the valves due to sodium precipitation, the control system can automatically cause the recharger to periodically rinse the system with water at preset times. The control system can also cause the recharger to rinse the system with water between chemicals. After pumping a first fluid, such as a disinfectant, through the recharging flow path, the control system can pump water through the recharging flow path to rinse the recharging flow path before pumping brine or base. The control system can cause the recharger to rinse the system any time the chemicals are changed.

The pressure, flow rate, and conductivity of the fluid are determined with various sensors located in the recharging flow paths, each of which is in communication with the control system. The sensors transmit data to the control system for determination of the system state. Based on the data received from the sensors, the control system determines whether the pressure, flow rate, and conductivity are within predetermined ranges by comparing the measured parameters from the sensors with predetermined ranges stored by the control system. The described alerts indicating a leak, an occlusion, a pump failure, or a chemical-run out can be generated by the system in any fashion, including through an audio or visual alert, or combinations thereof. The system can generate an audio alert by activating an alarm or tone informing a user that a leak, occlusion, pump failure, or chemical run-out has occurred or is occurring. The system can generate a visual alert by activating a warning light or creating a text based message on a user interface.

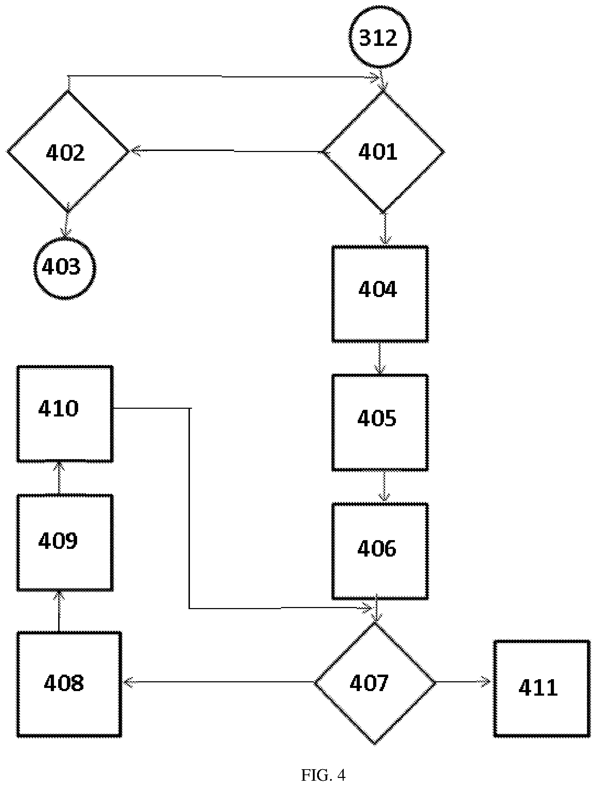

FIG. 4 illustrates the control algorithms used during the disinfection process. After receiving instructions to continue the recharging process in step **312,** the system begins to monitor the zirconium phosphate and zirconium oxide effluent line conductivity in step **401.** Once the disinfectant has completely filled the sorbent modules the conductivity of the effluent should approach the conductivity of the disinfectant solution used for disinfection. If the conductivity is outside of some predetermined range of the expected conductivity, the system determines how much time has elapsed from the beginning of the disinfection process in step **402.** If the time elapsed exceeds a predetermined threshold, the system can stop the recharging process in step **403** by stopping the pumps. The predetermined threshold is a range of time based on the amount of time expected to elapse before the sorbent module is completely filled with disinfectant. The expected amount of time varies with the size of the sorbent module and the disinfectant solution flow rate, and as such, the predetermined threshold can also vary. Because the zirconium phosphate sorbent module is generally larger than the zirconium oxide sorbent module, the predetermined threshold is higher for the zirconium phosphate recharging flow path than the zirconium oxide recharging flow path. If the predetermined threshold has not been reached, the system can continue to monitor the effluent conductivity in step **401.**

As described, the disinfectant can be sequestered within the sorbent modules to ensure complete disinfection. Once the system determines the effluent conductivity is within a predetermined range of the disinfectant solution conductivity, the pump is stopped and valves closed in step **404** to sequester the disinfectant in the sorbent module. In step **405** the zirconium phosphate and zirconium oxide control systems send a message to the user interface system, and optionally displayed to the user, indicating the disinfection process has begun. In step **406,** a timer is started to track the amount of time the disinfectant is sequestered within the sorbent module. The recharging control system tracks the time elapsed and determines if the disinfection process is complete in step **407.** The disinfectant can be sequestered in the sorbent module for any length of time sufficient to disinfect the sorbent module, including between 5 and 20 minutes. Other suitable times are contemplated by the invention. Once the timer reaches the sequester time, the system can send a message indicating the disinfection process is complete in step **411.**

As described, the disinfectant solution used for disinfection can be a peracetic acid solution. During disinfection, the peracetic acid solution may generate carbon dioxide, which needs to be vented from the sorbent modules. The carbon dioxide is vented at set periods of time to prevent the buildup of excess pressure. In step **407,** the system determines whether the set period of time has elapsed, indicating the carbon dioxide should be vented. If the set period of time has elapsed, the system opens the valves and starts the pumps to vent the modules in step **408.** In step **409,** the disinfectant solution is pumped through the module for a set length of time to ensure venting of the carbon dioxide. The pump is shut off and the valves closed in step **410,** and the system continues to monitor the timer in step **407.**

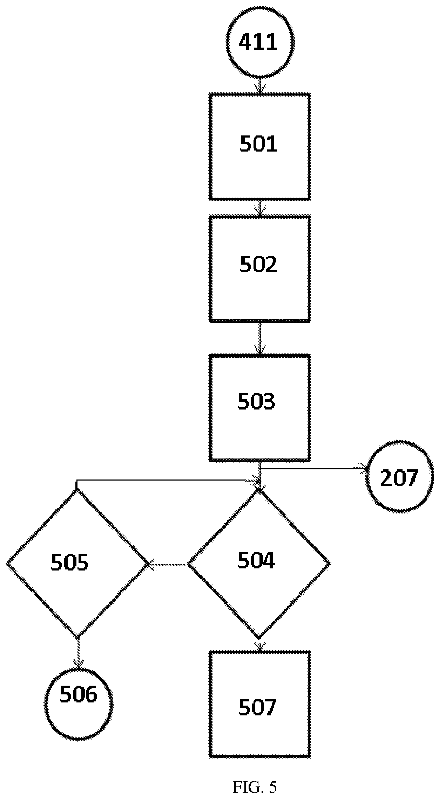

FIG. 5 illustrates the control algorithms for rinsing the modules after disinfection. After the system indicates that the disinfection of the zirconium phosphate and zirconium oxide modules are complete in step **411,** the control system begins the rinse sequence in step **501.** In step **502,** the recharger flow paths are configured to pump water from a water source through the reusable modules. The water is pumped through the recharging flow paths in step **503.** Concurrently, the control system conducts the chemical and system test sequence **207,** as illustrated in FIG. 3. After conducting the chemical and system tests, the control system monitors the conductivity of the effluent of the zirconium phosphate and zirconium oxide modules in step **504** and determines whether the conductivity is within a pre-set range of the expected effluent conductivity. After the rinsing sequence is complete, the conductivity of the effluent should approach that of water. If the conductivity is not within a predetermined range of the expected conductivity, the system determines the time elapsed from the beginning of the rinse sequence in step **505.** If the time elapsed exceeds a predetermined threshold, the system stops the recharging process in step **506** by stopping the pumps. The predetermined threshold is a range of time based on the amount of time expected to elapse before the sorbent module is completely rinsed of disinfectant. The expected amount of time varies with the size of the sorbent module and the water flow rate, and the predetermined threshold can also vary. Because the zirconium phosphate sorbent module is generally larger than the zirconium oxide sorbent module, the predetermined threshold is higher for the zirconium phosphate recharging flow path than the zirconium oxide recharging flow path. If the predetermined threshold has not been reached, the system continues to monitor the effluent conductivity in step **504.** Once the system determines that the conductivity of the effluent is within the predetermined range, the system generates a message indicating the rinse process is complete in step **507.**

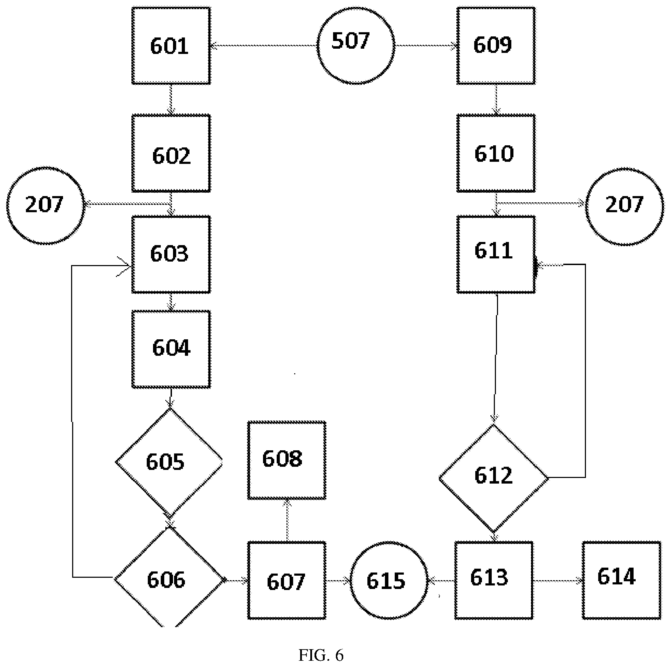

After generating messages that the rinse process is complete in step **507,** the system begins the first stage of the recharging process as illustrated in FIG. 6. The control system sends a message to the zirconium phosphate control system to begin the recharging process in step **601.** As described, recharging of zirconium phosphate requires the use of a brine solution. During zirconium phosphate recharging, potassium, calcium, magnesium, and ammonium ions bound to the zirconium phosphate must be replaced by hydrogen and sodium ions. The final ratio of hydrogen to sodium ions on the recharged zirconium phosphate is determined by the pH, buffer capacity, and sodium concentration of the brine solution used in the recharging process. The brine solution can be a mixture of sodium chloride, sodium acetate, and acetic acid. In one non-limiting brine solution, the sodium chloride concentration can be between 2.5M and 4.9M, the sodium acetate concentration can be between 0.3M and 1.1M, and acetic acid concentration can be between 0.2M and 0.8M. The zirconium phosphate control system begins to pump brine solution from a brine source through the zirconium phosphate recharging flow path in step 602. As the system pumps brine solution through the zirconium phosphate module, the system performs a chemical and system check in step 207, as illustrated in FIG. 3. In step 603, the system continues to pump brine solution through the zirconium phosphate recharging flow path and calculates the volume of brine solution pumped into the zirconium phosphate module. In step 604, a heater can be operated to heat the brine solution, as recharging of zirconium phosphate can become more efficient at elevated temperatures. In step 605, the system determines the temperature of the brine solution entering the zirconium phosphate module to ensure the brine solution is heated to the proper temperature. In step 606, the system begins monitoring the conductivity of the effluent of the zirconium phosphate module with a conductivity sensor to determine when brine solution begins to exit the zirconium phosphate module. If the conductivity does not show brine present in the effluent, the system continues to pump brine through the zirconium phosphate flow path in step **603.** Once brine is detected in the zirconium phosphate effluent, the system stops calculating the amount of brine pumped in step **607.** A message is sent to the control system indicating the first stage of the zirconium phosphate recharging process is complete, as well as the total amount of brine pumped through the zirconium phosphate, in step **615.** At the same time, the zirconium phosphate control system enters a wait state in step **608,** wherein the pumps are stopped until the system indicates that the first stage of the zirconium oxide recharging process is complete.

Concurrently with the zirconium phosphate recharging, the system also sends a message to the zirconium oxide control system to begin the recharging process in step **609.** As described, recharging of zirconium oxide requires the use of a base solution. The solution can be any suitable basic solution capable of replacing phosphate and other anions bound to the zirconium oxide with hydroxide ions. The hydroxide base can be any suitable base such as sodium hydroxide. One non-limiting example is sodium hydroxide having a concentration between 0.8M and 1.2M. In step **610,** the base solution is pumped from a base source through the zirconium oxide recharging flow path to the zirconium oxide module. The system performs a chemical and system test in step **207,** as illustrated in FIG. 3. In step **611,** the system continues to pump the base solution through the zirconium oxide recharging flow path, and calculates the amount of base solution pumped. In step **612,** the system monitors the conductivity of the zirconium oxide effluent and determines whether the effluent contains any base. If the system cannot detect the base solution, the system continues to pump the base solution through the zirconium oxide recharging flow path in step **611.** Once base is detected in the zirconium oxide effluent, the system stops calculating the amount of base solution pumped in step **613.** A message is sent to the control system indicating the first stage of the zirconium oxide recharging process is complete, as well as the total amount of base pumped through the zirconium oxide, in step **615.** At the same time, the zirconium oxide control system enters a wait state in step **614,** wherein the pumps are stopped until the system indicates that the first stage of the zirconium phosphate recharging process is complete.

The zirconium phosphate requires an acidic brine solution for recharging, while the zirconium oxide requires a basic solution. The dual recharging system described herein allows for inline neutralization of the zirconium phosphate effluent having an acidic pH with the zirconium oxide effluent having a basic pH. The first stage of recharging each module is halted as illustrated in FIG. 6, to ensure that the processes for inline neutralization are synchronized in each recharging flow path. One of skill in the art will understand that if inline neutralization is not required, the system does not need to synchronize the zirconium phosphate and zirconium oxide recharging processes, and the system need not enter a wait state as illustrated in FIG. 6.

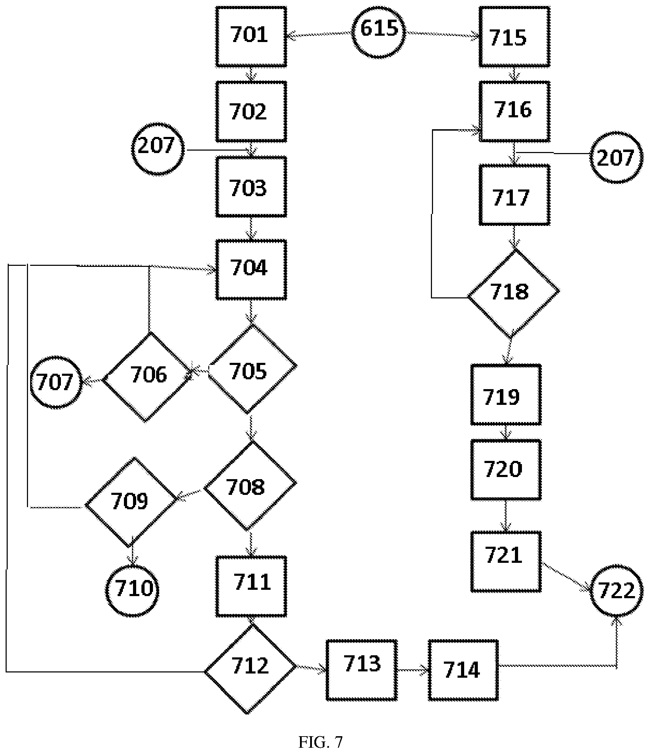

After receiving the messages that the first stage of recharging is complete in step **615,** the system can begin the second stage of the recharging process as illustrated in FIG. 7. In step **701** the control system sends a message to the zirconium phosphate control system to begin the second stage of the recharging process. The zirconium phosphate recharging flow path is configured to pump brine through the zirconium phosphate module in step **702.** After the system begins to pump brine through the zirconium phosphate recharging flow path, the system performs a chemical and system test in step **207,** as illustrated in FIG. 3. In step **703,** the system begins to calculate the amount of brine solution pumped through the zirconium phosphate recharging flow path. In step **704,** the heater is activated to heat the brine solution. In step **705,** the system determines whether the temperature of the brine solution at the inlet of the zirconium phosphate module is above some pre-set temperature with a temperature sensor in communication with the control system. The pre-set temperature can be any elevated temperature, including between 65°C and 95°C. If the brine solution has not reached the desired temperature, the system determines the length of time that has elapsed since the heater was activated in step **706.** If the length of time is above some preset time period, the system can be stopped in step **707,** and an alert generated indicating a possible heater failure. If the preset time has not been reached, the system continues to pump brine solution. In step **708,** the system determines whether the temperature at the zirconium phosphate sorbent module outlet is above some preset temperature, generally lower than the inlet preset temperature. The pre-set temperature for the zirconium phosphate sorbent module outlet can be any elevated temperature, including between 60°C and 80°C. If the zirconium phosphate effluent has not reached the preset temperature, the system determines the length of time that has elapsed since the heater was activated in step **709.** If the length of time is above some preset time period, the system can be stopped in step **710,** and an alert generated indicating a possible heater failure. If the preset time has not been reached, the system continues to pump brine solution. In step **711,** the system calculates the amount of brine necessary for recharging the zirconium phosphate module to ensure complete recharging at the temperature measured at the zirconium phosphate sorbent module outlet. Because zirconium phosphate recharging is more efficient at higher temperatures, the amount of brine necessary for recharging the zirconium phosphate module varies with the temperature. The control system can calculate the amount of brine necessary for recharging the zirconium phosphate module through mathematical algorithms providing a relationship between temperature and the amount of brine necessary for recharging. Alternatively, the control system can use a look-up table populated with previous results for recharging at various temperatures. The control system can determine the temperature at the outlet of the zirconium phosphate module, and find an amount of brine necessary for recharging the zirconium phosphate module in the look-up table. In step **712,** the system determines the total amount of brine pumped using a flow sensor in communication with the control system, and determines whether the total amount of brine is at least the calculated amount. If the total amount of brine pumped is less than the calculated amount, the system continues to pump brine. Once the total amount of brine pumped is at least the calculated amount, the control system determines whether the second stage of the zirconium oxide recharging process has been complete. Once the second stage of the zirconium oxide recharging process is complete, the zirconium phosphate recharging flow path is placed into a wait state in step **713** and the pumps and heater are stopped. If the second stage of the zirconium oxide recharging process is not complete, the control system will continue to pump brine through the zirconium phosphate. The total amount of brine pumped through the zirconium phosphate module is sent to the control system in step **714,** and a message is generated indicating the second stage of recharging is complete in step **722.**

In step **715,** the control system sends a message to the zirconium oxide control system to begin the second stage of the recharging process. The zirconium oxide recharging flow path is configured to pump a base solution through the zirconium oxide module in step **716.** After the system begins to pump the base solution through the zirconium oxide recharging flow path, the system performs a chemical and system test in step **207,** as illustrated in FIG. 3. In step **717,** the system begins calculating the total amount of base solution pumped through the zirconium oxide module. As described, the control system can calculate a neutralization ratio based on the brine solution and the base solution, wherein the neutralization ratio is the ratio of base solution to brine solution necessary for complete neutralization of each solution. The control system can calculate the neutralization ratio based on the known pH of the brine and base solutions. In step **718,** the system, using the neutralization ratio, determines whether the amount of base solution pumped through the zirconium oxide module is the proper amount for neutralization of the brine solution pumped through the zirconium phosphate module to generate a fluid within a predetermined pH range safe for disposal. In any embodiment, the predetermined pH range can be between 5 and 9. If additional base solution is necessary for neutralization of the brine solution, the system continues to pump base solution through the zirconium oxide recharging flow path in step **716.** The volume of base passed through the zirconium oxide recharging flow path in the second stage should be greater than or equal to the volume of base required to recharge the zirconium oxide, minus the total amount of brine pumped in the first stage of the zirconium phosphate recharging process, divided by the neutralization ratio, minus the total amount base pumped in the first stage of the zirconium oxide recharging process. Once enough base solution has been pumped through the zirconium oxide module to neutralize the brine solution pumped through the zirconium phosphate module, the control system determines whether the second stage of the zirconium phosphate recharging process is complete. If the second stage to the zirconium phosphate recharging process is complete, the system enters a wait state in step **719,** and the pumps deactivated in step **720.** If the second stage of the zirconium phosphate recharging process is incomplete, the system continues to pump base through the zirconium oxide. In step **721,** the total amount of base solution pumped is sent to the control system. In step **722,** a message is generated indicating the second stage of recharging is complete.

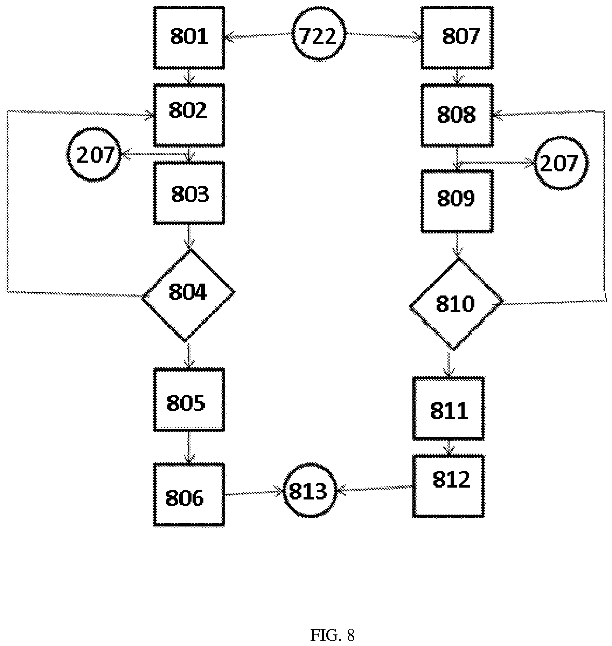

After recharging the zirconium phosphate as illustrated in FIG.'s 6-7, the system rinses the zirconium phosphate module to remove any remaining brine solution. FIG. 8 illustrates the control algorithms used in the first stage of the rinse process. After receiving the messages that the second stage of recharging is complete in step **722,** the control system sends a message to the zirconium phosphate control system to begin the rinse process in step **801.** In step **802,** the zirconium phosphate recharging flow path is configured to pump water through the zirconium phosphate module. After the system begins to pump water, a chemical and system test is conducted in step **207,** as illustrated in FIG. 3. In step **803,** the volume of water pumped through the zirconium phosphate module is calculated with a flow sensor in communication with the control system at the zirconium phosphate sorbent module outlet. In step **804,** the system determines whether the total amount of water pumped through the zirconium phosphate module is equal to the total amount of brine pumped in the first stage of the recharging process, minus the total amount of base pumped in the first stage of the recharging process times the neutralization ratio. If the volume of water pumped is less than the calculated volume, the system continues to pump water in step **802.** Once the volume of water pumped reaches the calculated volume, the system enters a wait state in step **805,** and the total amount of water pumped is transmitted to the recharger control system in step **806.** The zirconium phosphate recharging control system generates a message indicating the first stage of rinsing is complete in step **813.**

In step **807,** the control system sends a message to the zirconium oxide control system to begin the third stage of the zirconium oxide recharging process. In step **808,** the zirconium oxide recharging flow path is configured to pump base solution through the zirconium oxide recharging flow path. Base is pumped through the zirconium oxide recharging flow path in step **808** while the first stage of the zirconium phosphate rinse process begins to ensure neutralization of the brine solution in the zirconium phosphate effluent during the rinse process. The system conducts a chemical and system test in step **207,** as illustrated in FIG. 3. In step **809,** the volume of base solution pumped through the zirconium oxide module is calculated with a flow sensor in communication with the control system positioned at the zirconium oxide sorbent module outlet. In step **810,** the system determines whether the volume of base solution pumped is equal to the volume of brine solution pumped in the first stage of the recharging process divided by the neutralization ratio, minus the volume of base pumped in the first stage of the recharging process. If the volume of base pumped in the third stage of the recharging prcoessprocess is less than this calculated volume, the system continues to pump base solution in step **808.** Once the volume of base pumped in the third stage of the recharging process reaches the calculated volume, the system enters a wait state in step **811,** and the total volume of base pumped is sent to the control system in step **812.** A message indicating the third stage of the recharging process is complete is generated in step **813.**

After receiving the messages that the first stage of the zirconium phosphate rinse process, and the third stage of the zirconium oxide recharging process are complete in step **813,** the control system sends a message to the zirconium phosphate control system to begin the second stage of the rinse process in step **901.** In step **902,** the zirconium phosphate recharging flow path is configured to pump water through the zirconium phosphate module. After the system begins to pump water, a chemical and system test is conducted in step **207,** as illustrated in FIG. 3. In step **903,** the volume of water pumped through the zirconium phosphate module in the second stage of the rinse process is calculated. In step **904,** the system determines whether the total amount of water pumped through the zirconium phosphate module in the second stage of the rinse process is equal to the total amount of base pumped in the first stage of the recharging process times the neutralization ratio. If the volume of water pumped is less than the calculated volume, the system continues to pump water in step **902.** Once the volume of water pumped reaches the calculated volume, the system enters a wait state in step **905,** and the total amount of water pumped is transmitted to the recharger control system in step **906.** The zirconium phosphate recharging control system generates a message indicating the second stage of rinsing is complete in step **913.**

In step **907,** the control system sends a message to the zirconium oxide control system to begin the rinse process. In step **908,** the zirconium oxide recharging flow path is configured to pump water through the zirconium oxide recharging flow path. The system conducts a chemical and system test in step **207,** as illustrated in FIG. 3. In step **909,** the volume of water pumped through the zirconium oxide module is calculated with a flow sensor in communication with the control system positioned at the zirconium oxide sorbent module outlet. In step **910,** the system determines whether the volume of water pumped is equal to the volume of base solution pumped in the first stage of the recharging process. If the volume of water pumped in the rinse process is less than this calculated volume, the system continues to pump water in step **908.** Once the volume of water pumped in the rinse process reaches the calculated volume, the system enters a wait state in step **911,** and the total volume of water pumped is sent to the control system in step **912.** A message indicating the second stage of the rinse process is complete is generated in step **913.**

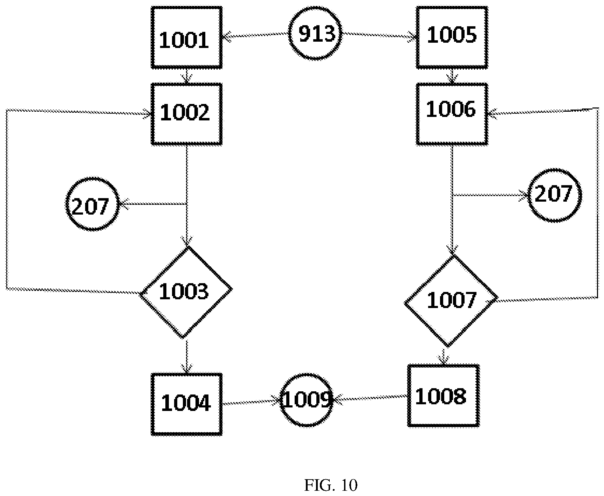

FIG. 10 illustrates the third stage of the rinse process. After receiving the messages the second stage of the rinse process is complete in step **913,** the recharger control system sends a message to the zirconium phosphate control system to begin the third stage of the rinse process in step **1001.** The zirconium phosphate recharging flow path is configured to pump water through the zirconium phosphate module in step **1002.** After starting a pump to pump water, the system performs a chemical and system check in step **207,** as illustrated in FIG. 3. In step **1003,** the conductivity of the zirconium phosphate effluent is measured with a conductivity sensor in communication with the control system. If the conductivity is outside of a predetermined range, the system continues to pump water in step **1002.** The predetermined range can be the conductivity of water at the same temperature as the zirconium phosphate effluent, such as at 40°C, indicating the brine solution has been completely rinsed from the module. Once the system determines that the brine has been completely rinsed, the zirconium phosphate control system enters a wait state in step **1004,** and a message is sent to the recharging control system that the rinse process is complete in step **1009.**

In step **1005,** the recharger control system sends a message to the zirconium oxide control system to begin the second stage of the rinse process. In step **1006** the zirconium oxide recharging flow path is configured to pump water through the zirconium oxide module. After starting a pump to pump water, the system performs a chemical and system check in step **207,** as illustrated in FIG. 3. In step **1007,** the conductivity of the zirconium oxide effluent is measured with a conductivity sensor in communication with the control system. If the conductivity is outside of a predetermined range, indicating some base solution remains in the effluent, the system continues to pump water in step **1006.** Once the system determines that the base solution has been completely rinsed, the zirconium oxide control system enters a wait state in step **1008,** and a message is sent to the recharging control system that the rinse process is complete in step **1009.** After the control system receives both messages in step **1009,** the recharging process is complete and the zirconium phosphate and zirconium oxide modules can be reused in dialysis.

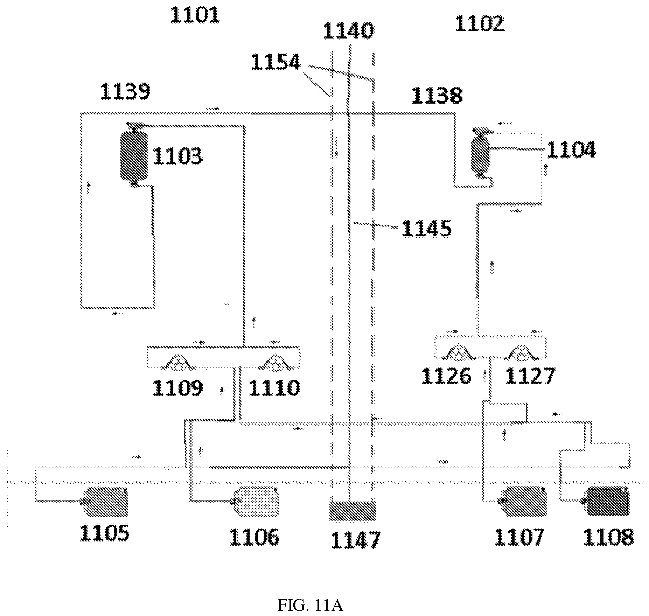

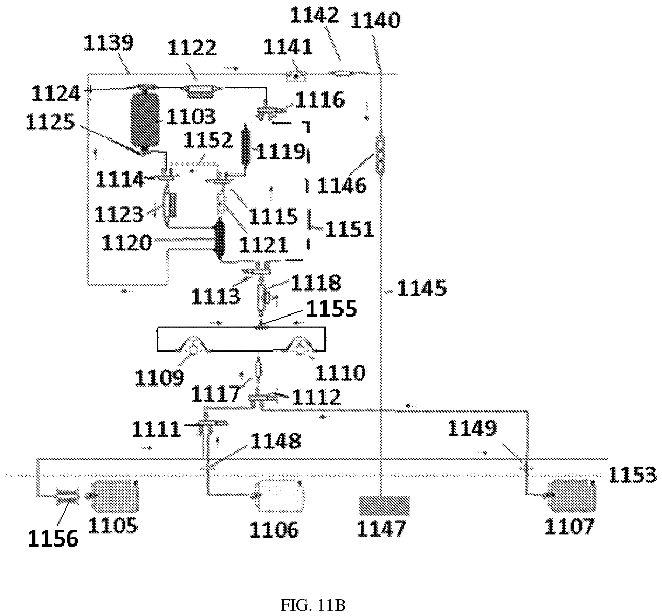

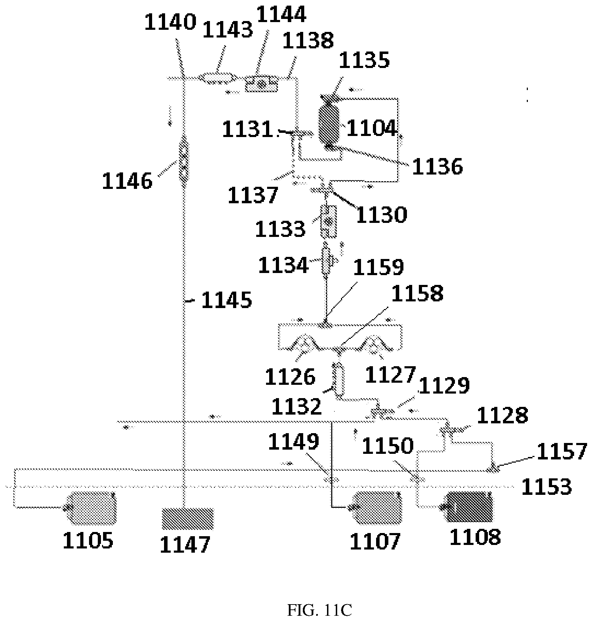

To recharge the sorbent materials, fluids from fluid sources are passed through the sorbent modules. The flow paths of the invention can be arranged as shown in FIG.'s 11A-C. FIG. 11A is a generalized view of a recharging flow path, with details shown in FIG.'s 11B and 11C. The recharging flow path can be divided into a zirconium phosphate recharging flow path **1101** containing the zirconium phosphate module **1103** and a zirconium oxide recharging flow path **1102** containing zirconium oxide module **1104.** Details of the zirconium phosphate recharging flow path **1101** on the zirconium phosphate side of line **1154** are illustrated in FIG. 11B, while details of the zirconium oxide recharging flow path **1102** on the zirconium oxide side of line **1154** are illustrated in FIG. 11C. Although a dual cartridge recharger system is shown, single, two or more multiple cartridge recharger systems are envisioned. Any one of the recharger cartridge systems can be linked together to share resources for recharging the sorbent cartridge and can be adapted for large scale use. Similarly, the linked rechargers can be scaled down as demand for recharging decreases. The modular recharging set-up having more or less rechargers based on demand can be advantageously used where required.

In FIG. 11A, a zirconium phosphate recharging flow path **1101** and a zirconium oxide recharging flow path **1102** have a water source **1105,** a brine source **1106,** a disinfectant source **1107,** and a base source **1108.** The brine source **1106,** disinfectant source **1107,** and/or base source **1108** can be a column containing a dry bed of the brine, disinfectant, and/or base components. Alternatively, a powdered source of the brine, disinfectant, and/or base components can be used. The dry bed or powdered source can be dissolved with an aqueous solution. A static mixer (not shown) can mix the single line coming through the column prior to entering the zirconium phosphate module **1103** or zirconium oxide module **1104.** Recharging the zirconium phosphate in a zirconium phosphate module **1103** requires water, brine, and disinfectant. The water source **1105,** the brine source **1106,** and the disinfectant source **1107** are fluidly connected to the zirconium phosphate recharging flow path **1101.** Similarly, recharging zirconium oxide module **1104** in zirconium oxide recharging flow path **1102** requires water, base, and disinfectant. The water source **1105,** the disinfectant source **1107,** and the base source **1108** are fluidly connected to the zirconium oxide recharging flow path **1102.** The zirconium phosphate recharging flow path **1101** and zirconium oxide recharging flow path **1102** can be operated simultaneously or independently. Disinfectant source **1107** can contain any type of disinfectant compatible with zirconium phosphate and zirconium oxide that is capable of disinfecting the reusable sorbent modules. In any embodiment, the disinfectant source **1107** can contain peracetic acid. In any embodiment, the peracetic acid can be a solution of between 0.5 % and 2% peracetic acid in water. The brine source **1106** can have an acid, a base, and a sodium salt.

During zirconium phosphate recharging, potassium, calcium, magnesium, and ammonium ions bound to the zirconium phosphate must be replaced by hydrogen and sodium ions. The final ratio of hydrogen to sodium ions on the recharged zirconium phosphate can be determined by the pH and sodium concentration of the brine solution used in the recharging process. The brine source **1106** can be a mixture of sodium chloride, sodium acetate, and acetic acid. In one non-limiting brine solution, the sodium chloride concentration can be between 2.5 M and 4.9 M, the sodium acetate concentration can be between 0.3 M and 1.1 M, and acetic acid concentration can be between 0.2 M and 0.8 M. The water source **1105** can contain any type of water, including deionized water. To recharge the zirconium phosphate in the zirconium phosphate module **1103,** the disinfectant from disinfectant source **1107** can flow to the zirconium phosphate module **1103** to disinfect the zirconium phosphate module **1103.** Fluid from the disinfectant source **1107** can flow to valve **1112** in the zirconium phosphate recharging flow path **1101.** Zirconium phosphate pumps **1109** and **1110** provide a driving force to pump the fluid through the zirconium phosphate recharging flow path **1101.** Use of two or more separate pumps can reduce wear on the pumps. Correspondingly, smaller pumps can be used. The two or more pumps can provide in-line mixing and intermittent pumping so at any given time, a single pump can pump fluid through the zirconium phosphate recharging flow path **1101.** The two pumps can be used simultaneously or independently. The two or more pumps can provide fluid line mixing of one or more separate fluid streams when used simultaneously. The two or more pumps can operate asynchronously but used concurrently. For example, a first pump can operate for a time and a second pump remain off, then the first pump shut off with the second pump turning on. Multiple pumps at various timed pumping stages are envisioned as described herein. One of skill in the art will understand that a single zirconium phosphate pump can also accomplish the described pump functions.

Zirconium phosphate pumps **1109** and **1110** can pump fluid from disinfectant source **1107** through valve **1112** and valve **1113.** Fluid can be pumped through three-way junction **1155** to valve **1116** and into zirconium phosphate module **1103** through zirconium phosphate module inlet **1124.** The illustrated junctions combine the inlet chemical or water pumped by the two pumps such that higher flow rates can be achieved. During filling, fluid inside zirconium phosphate module **1103** can be forced through zirconium phosphate module outlet **1125** and into zirconium phosphate module effluent line **1139.** The disinfectant can be sequestered in the zirconium phosphate module **1103** to ensure disinfection. Heater **1119** upstream of the zirconium phosphate module **1103** can heat the disinfectant because disinfection can become more efficient at elevated temperatures. After disinfection, zirconium phosphate module **1103** can be rinsed using water from water source **1105.** Zirconium phosphate pumps **1109** and **1110** can pump water from water source **1105** through valves **1111** and **1112** to valve **1113.** The water can then be pumped through valves **1115** and **1116** through the zirconium phosphate module **1103** through zirconium phosphate module inlet **1124,** out zirconium phosphate module outlet **1125** and into zirconium phosphate module effluent line **1139.** Water can be pumped through the zirconium phosphate module **1103** until all of the disinfectant is removed.

Fluid from brine source **1106** can be pumped through the zirconium phosphate module **1103** to load the zirconium phosphate module **1103** with the proper ratio of sodium and hydrogen ions. Zirconium phosphate pumps **1109** and **1110** can pump fluid from brine source **1106** to valve **1111.** The brine can follow the same pathway as the water through zirconium phosphate module **1103** and into zirconium phosphate module effluent line **1139.** Heater **1119** upstream of the zirconium phosphate module **1103** can heat brine because recharging can become more efficient at elevated temperatures. Heat exchanger **1120** can lessen the load on heater **1119.** One or more heat exchangers and one or more heaters can be used. The heat exchanger **1120** can be fluidly connected to zirconium phosphate module effluent line **1139** and to zirconium phosphate module inlet **1124** upstream of heater **1119.** The heated fluid exiting the zirconium phosphate module **1103** in zirconium phosphate module effluent line **1139** can heat the incoming brine solution in heat exchanger **1120.** The heat exchanger **1120** can have at least a first chamber and a second chamber. Fluid in the zirconium phosphate inlet lines can pass through the first chamber of the heat exchanger **1120,** and fluid in the zirconium phosphate effluent line **1139** can pass through the second chamber of the heat exchanger **1120.** The increased temperature of the zirconium phosphate effluent in the second chamber can heat the fluid in the zirconium phosphate inlet lines in the first chamber. The zirconium phosphate module **1103** can be rinsed again by pumping water through the zirconium phosphate module **1103.** A static mixer (not shown) can be positioned upstream of the zirconium phosphate module **1103** and mix the solutions prior to entering the zirconium phosphate module **1103.**

Various sensors in the zirconium phosphate module recharging flow path **1101** can ensure proper concentrations and temperatures as shown in FIG. 11B. For example, conductivity sensor **1117** can ensure the incoming water contains no defined level of ions that may interfere with the recharging process, and that the brine solution and disinfectant solution are at a desired concentration. Conductivity sensor **1117** can also ensure sufficient rinsing has occurred to remove brine and disinfectant solution. Pressure sensor **1118** can monitor pressure in the zirconium phosphate inlet lines to ensure there are no occlusions or leaks and that the inlet pressures are in an acceptable range. Temperature sensor **1122** can ensure the brine solution is at the proper temperature before entering zirconium phosphate module **1103** and to control heater **1119.** Temperature sensor **1123** can be placed in zirconium phosphate module effluent line **1139** to monitor the temperature of the effluent which can be controlled by heat exchanger **1120** and heater **1119.** A flow sensor **1121** can monitor the flow rates of the fluids in the zirconium phosphate recharging flow path **1101** and control zirconium phosphate pumps **1109** and **1110.** One of skill in the art will understand that alternative arrangements of sensors can be used in FIG. 11B and that one or more additional sensors can be added. Further, the sensors can be placed at any appropriate position in the zirconium phosphate recharging flow path **1101** to determine fluid parameters at various locations throughout the zirconium phosphate recharging flow path **1101.**

Zirconium phosphate module bypass line **1152** fluidly connects valve **1115** to valve **1114** in the zirconium phosphate effluent line **1139.** Valves **1115** and **1116** can be controlled to direct fluid through the zirconium phosphate module bypass line **1152** and into zirconium phosphate effluent line **1139.** The dual flow path aspect of the recharging flow path depicted in FIG. 11A can neutralize the effluent from both the zirconium phosphate module **1103** and zirconium oxide module **1104** by mixing the acidic effluent from the zirconium phosphate module **1103** with the basic effluent from zirconium oxide module **1104.** If only zirconium oxide module **1104** is being recharged using the flow path of FIG. 11C, the zirconium phosphate module bypass line **1152** can be utilized to direct fluid from the brine source **1106** to the zirconium phosphate effluent line **1139** to neutralize the zirconium oxide effluent without the need to simultaneously recharge a zirconium phosphate module **1103.** Alternatively, zirconium phosphate module inlet **1124** can directly connect to zirconium phosphate module outlet **1125.** The zirconium phosphate recharging flow path **1101** can include a rinse loop **1151** to fluidly connect valve **1113** upstream of the heater **1119** and heat exchanger **1120** to valve **1116,** bypassing heater **1119** and heat exchanger **1120.** The rinse loop **1151** can rinse brine solution from the zirconium phosphate module **1103.** By bypassing heater **1119** and heat exchanger **1120** through rinse loop **1151,** the zirconium phosphate module **1103** can be cooled faster.

To recharge the zirconium oxide module **1104,** disinfectant from disinfectant source **1107** can be first pumped to the zirconium oxide module **1104** to disinfect the zirconium oxide module **1104.** Fluid from the disinfectant source **1107** can be pumped to valve **1129** in the zirconium oxide recharging flow path **1102.** Zirconium oxide pumps **1126** and **1127** can pump fluid through the zirconium oxide recharging flow path **1102.** As described, a single zirconium oxide pump is contemplated as an alternative to the dual pump system in FIG. 11. Also, two or more zirconium oxide pumps are contemplated. The two or more zirconium oxide pumps can provide fluid line mixing of one or more separate fluid streams when used simultaneously. The two or more pumps can be asynchronous but used concurrently. For example, a first pump can operate for a time and a second pump remain off, then the first pump shut off with the second pump turning on. Multiple pumps at various timed pumping stages are envisioned as described herein. Zirconium oxide pumps **1126** and **1127** pump fluid from disinfectant source **1107** through valve **1129** to valve **1130.** The fluid flows to the zirconium oxide module **1104** through zirconium oxide module inlet **1135.** During filling, fluid inside zirconium oxide module **1104** can flow through zirconium oxide module outlet **1136** and into zirconium oxide module effluent line **1138.** The disinfectant can be sequestered in zirconium oxide module **1104** to ensure disinfection. The zirconium oxide module **1104** can then be flushed with water from water source **1105** after disinfection is completed. Zirconium oxide pumps **1126** and **1127** can pump water from water source **1105** through valves **1128** and **1129** and junction **1157** to valve **1130.** The fluid passes through junctions **1158** and **1159** to reach valve **1130.** The water can then be pumped to zirconium oxide module **1104** through zirconium oxide module inlet **1135** and out zirconium oxide module outlet **1136** and into zirconium oxide module effluent line **1138.** The zirconium oxide module **1104** can be flushed with any volume of water required to ensure the disinfectant is completely removed.

In FIG. 11C, zirconium oxide pumps **1126** and **1127** can pump fluid from base source **1108** through valve **1128** to zirconium oxide module **1104.** The base source **1108** can contain hydroxide ions to recharge zirconium oxide module **1104.** The hydroxide ions can flow through zirconium oxide module **1104** and into zirconium oxide module effluent line **1138.** The base source **1108** can be any suitable basic solution capable of replacing phosphate and other anions bound to the zirconium oxide with hydroxide ions. The hydroxide base can be any suitable base such as sodium hydroxide. One non-limiting example is sodium hydroxide having a concentration between 0.5M and 2.0M. Another non-limiting example is sodium hydroxide having a concentration at greater than 2% of the concentration of the recharging solution, including any concentration between 2% and 50%, 2% and 5%, 5% and 10%, 5% and 20%, 10% and 25%, 15% and 35%, 20% and 50%, 30% and 40%, or 40% and 50%.. A final rinse of the zirconium oxide module **1104** can be performed by pumping water through the zirconium oxide recharging flow path **1102** and zirconium oxide module **1104.** Zirconium oxide recharging flow path **1102** can also have a zirconium oxide module bypass line **1137** fluidly connecting valve **1130** in the zirconium oxide inlet line to valve **1131** in the zirconium oxide effluent line **1138.** Valves **1130** and **1131** can direct fluid through the zirconium oxide module bypass line **1137** and into zirconium oxide effluent line **1138.** Zirconium oxide module bypass line **1137** can convey fluid directly from the base source **1108** to the zirconium oxide effluent line **1138** to neutralize the zirconium phosphate effluent without the need to simultaneously recharge a zirconium oxide module **1104.** Alternatively, zirconium oxide module inlet **1135** can be fluidly connected to zirconium oxide module outlet **1136.** Multiple sensors can be included in the zirconium oxide recharging flow path **1102** to monitor fluid concentration. For example, conductivity sensor **1132** can monitor concentrations of the zirconium oxide recharging fluid; pressure sensor **1134** can monitor pressure in the zirconium oxide inlet line and to detect leaks or occlusions. Flow sensor **1133** can determine the flow rate of the fluid through the zirconium oxide inlet line and control zirconium oxide pumps **1126** and **1127.** A static mixer (not shown) can be positioned upstream of the zirconium oxide module **1104** and mix solutions prior to entering the zirconium oxide module **1104.** A heater and heat exchanger (not shown) can be positioned in the zirconium oxide recharging flow path **1102** to heat fluids prior to entering zirconium oxide module **1104.** Heating fluid in the zirconium oxide recharging flow path **1102** can reduce recharging times and allow disinfection with a base solution, such as sodium hydroxide. Heating the fluid also allows for reduced disinfection time with a disinfectant source. A zirconium oxide rinse loop (not shown) can also be included to bypass the heater and heat exchanger during flushing.

Effluent from zirconium phosphate recharging flow path **1101** can neutralize, either completely or in part, the effluent from zirconium oxide recharging flow path **1102,** and vice versa. Zirconium phosphate effluent line **1139** can be fluidly connected to zirconium oxide effluent line **1138** at an effluent line junction **1140** joining drain line **1145,** which fluidly connects to drain **1147.** Static mixer **1146** at or downstream of the effluent line junction **1140** can mix zirconium phosphate effluent with zirconium oxide effluent.

Zirconium phosphate effluent line **1139** and zirconium oxide effluent line **1138** can be connected to a common reservoir for storage and disposal of the combined effluent. The common reservoir receives and collects the zirconium phosphate and zirconium oxide effluents together. The collected effluents can be drained after appropriate volumes of each effluent have been added to achieve neutralization. A common reservoir can allow for neutralization of the zirconium phosphate and zirconium oxide effluents without synchronizing the recharging processes. A single common reservoir can be sized to support multiple recharge stations.

Alternatively, the two fluid streams may be mixed through fluid line mixing at the effluent line junction **1140.** Flow sensor **1141** and conductivity sensor **1142** can be placed in zirconium phosphate effluent line **1139** to measure the flow rate and composition of the zirconium phosphate effluent. Flow sensor **1144** and conductivity sensor **1143** can be positioned in the zirconium oxide effluent line **1138** to measure the flow rate and composition of the zirconium oxide effluent. Data from flow sensors **1141** and **1144** and conductivity sensors **1142** and **1143** can determine if the combined effluent in drain line **1145** is safe for disposal into a drain. One non-limiting example of safe is an effluent having a pH in the range of 5-9. Either zirconium phosphate effluent line **1139** or zirconium oxide effluent line **1138** can be connected simultaneously or independently to a waste reservoir (not shown) for disposal. Additional pH or conductivity sensors can be positioned downstream of the static mixer **1146** to monitor and ensure safe disposal. Drain line **1145** can also be connected to a common waste reservoir for storage and disposal of effluent. The common reservoir receives and collects the zirconium phosphate and zirconium oxide effluents together. The collected effluents can be drained after appropriate volumes of each effluent have been added to achieve neutralization. A common waste reservoir advantageously allows for neutralization of the zirconium phosphate and zirconium oxide effluents without synchronizing the recharging processes. Static mixer **1146** is unnecessary when a common reservoir is used.

Brine source **1106,** disinfectant source **1107**, and base source **1108** can have filter **1148,** filter **1149,** and filter **1150,** respectively to remove particulate matter. The one or more filters can remove particulate matter before fluid enters the zirconium oxide recharging flow path **1102** or zirconium phosphate recharging flow path **1101.** Water source **1105** can have microbial filter **1156** to remove microbes from the water before entering the flow paths. In FIG. 11C, the dashed line **1153** represents a recharger housing. The fluid sources can be external to the recharger housing and fluidly connected to the lines located inside of the recharger housing. Alternatively, the fluid sources described can instead be housed within the recharger.

During recharging, fluid can be passed through the zirconium phosphate module **1103** and/or the zirconium oxide module **1104** opposite to a flow direction used during dialysis. For example, zirconium phosphate module inlet **1124** can be the zirconium phosphate module outlet during dialysis, and zirconium phosphate module outlet **1125** can be the zirconium phosphate module inlet during dialysis in FIG. 11B. Similarly, zirconium oxide module inlet **1135** can be the zirconium phosphate module outlet during dialysis, and zirconium oxide module outlet **1136** can be the zirconium phosphate module inlet during dialysis. Pumping the recharging fluid through the modules in the opposite direction relative to dialysis can improve the efficiency of the recharging process.

The zirconium phosphate recharging flow path **1101** or zirconium oxide recharging flow path **1102** can independently recharge zirconium phosphate or zirconium oxide. For example, a single flow path fluidly connecting zirconium phosphate module **1103** of FIG. 11B via valve **1112** and valve **1113** to each of the water source **1105,** brine source **1106,** and disinfectant source **1107** can independently recharge the zirconium phosphate module **1103.** Similarly, a single flow path fluidly connecting zirconium oxide module **1104** of FIG. 11C via valve **1128** and valve **1129** to each of the water source **1105,** disinfectant source **1107,** and base source **1108** can independently recharge the zirconium oxide module **1104.**

The water source **1105,** brine source **1106,** disinfectant source **1107,** and base source **1108** can recharge one or more reusable sorbent module of various sizes. The amount of water, brine, disinfectant, and base depends on the concentration of each of the recharging solutions, the size of the reusable sorbent modules, the amount of cations/anions removed, and the flow rate used to pass the solutions through the reusable modules. The amount of brine solution required can depend on the temperature to which the brine solution is heated. For example, a brine solution having between 2.5 M and 4.9 M sodium chloride, between 0.3 M and 1.1 M sodium acetate, and between 0.2 M and 0.8 M acetic acid at between 65°C and 95°C requires between 4.2-6.2 L of brine to recharge a zirconium phosphate module containing between 2 kg and 3.2 kg of zirconium phosphate loaded with 2 to 3 moles of ammonium, calcium, magnesium and potassium. The brine solution should have a volume of at least between 4.2 and 6.2 L and delivered at a flow rate of between 100 and 300 mL/min. A single brine source can be connected to multiple rechargers, or can recharge multiple zirconium phosphate modules in a single recharger. The brine source can have a significantly larger volume from 1-100X or greater to ensure the brine source need not be refilled each time a zirconium phosphate is recharged. For a zirconium oxide module having between 220 and 340 g of zirconium oxide loaded with 200 mmols of phosphate, a base source having between 0.5 and 2.0 M sodium hydroxide and a flow rate between 30 and 150 mL/min requires between 1 and 4 L of base. The base source can be at least between 1 and 4 L in volume. For recharging multiple zirconium oxide modules, a larger base source can be used.

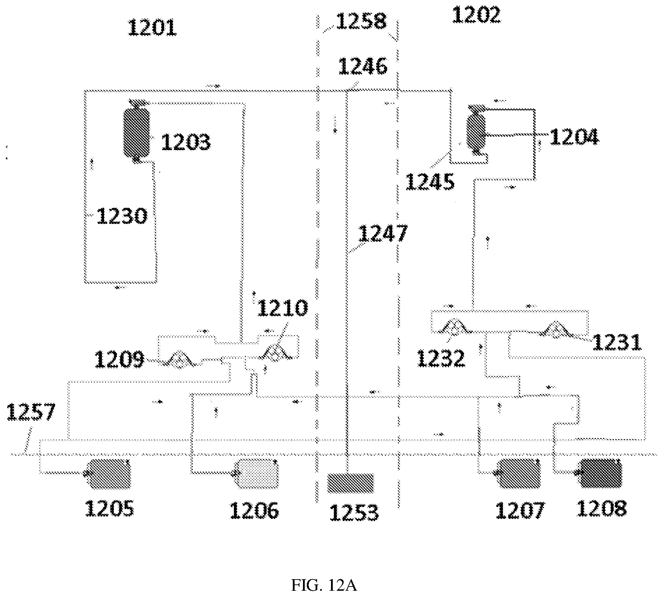

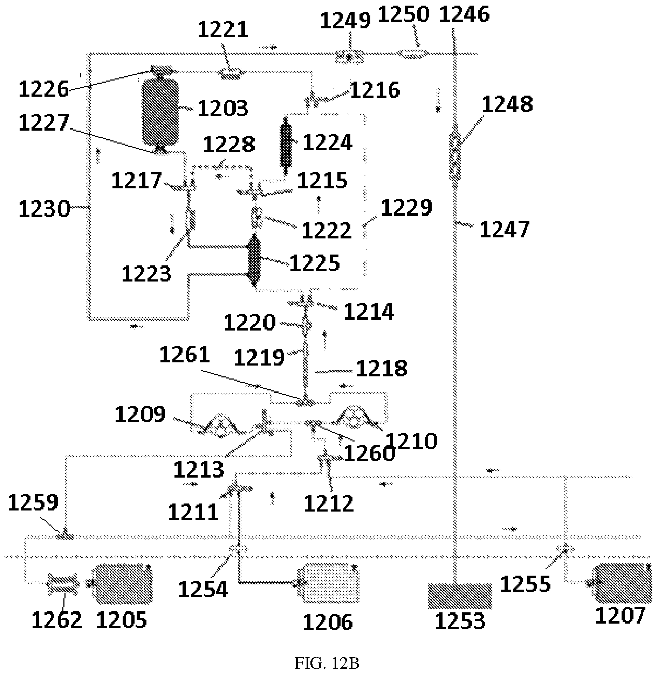

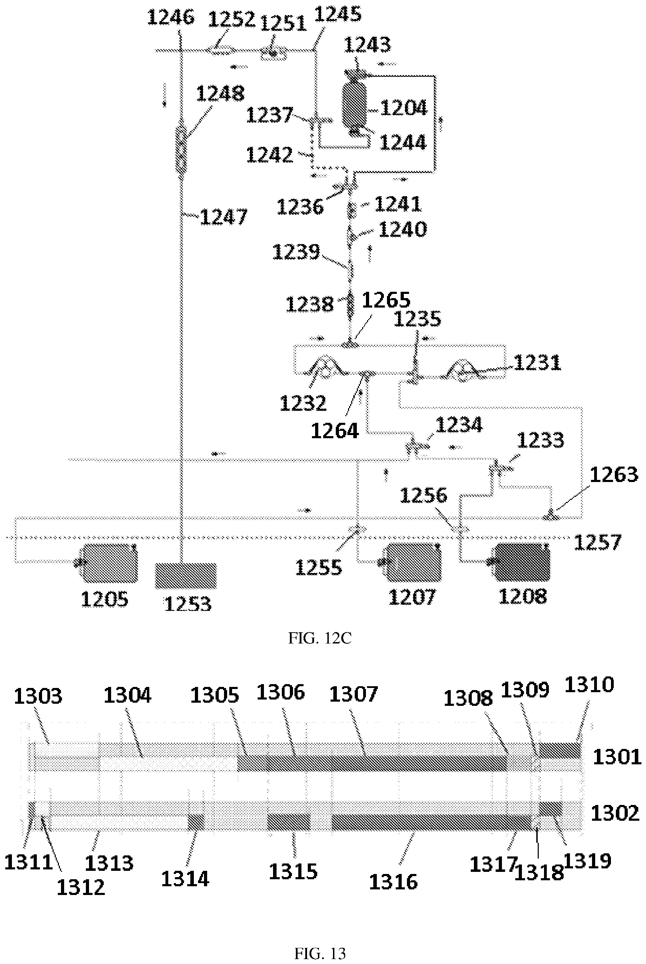

FIG. 12A is a generalized view of a recharging flow path having a zirconium phosphate recharging flow path **1201** containing a zirconium phosphate module **1203** and a zirconium oxide recharging flow path **1202** containing a zirconium oxide module **1204.** FIG. 12B illustrates a detailed view of zirconium phosphate recharging flow path **1201** on the zirconium phosphate side of line **1258,** and FIG. 12C illustrates a detailed view of zirconium oxide recharging flow path **1202** on the zirconium oxide side of line **1258.** The valves, pumps and static mixers illustrated in FIG.'s 12B and 12C allow for inline mixing of the recharging fluids. In FIG. 12A, the zirconium phosphate recharging flow path **1201** and/or zirconium oxide recharging flow path **1202** can be simultaneously or independently connected to a water source **1205,** a brine source **1206,** a disinfectant source **1207,** and a base source **1208.** Because recharging of the zirconium phosphate in a zirconium phosphate module **1203** can require water, brine, and disinfectant, and because recharging of zirconium oxide in zirconium oxide module **1204** can also require water, base, and disinfectant, the water source, **1205,** the brine source **1206,** and the disinfectant source **1207** can be jointly connected to the zirconium phosphate recharging flow path **1201,** and the water source **1205,** the disinfectant source **1207,** and the base source **1208** can be jointly connected to the zirconium oxide recharging flow path **1202.**

In FIG. 12A, zirconium phosphate recharging flow path **1201** and zirconium oxide recharging flow path **1202** can mix chemicals in-line to create the recharging solutions. Any one of disinfectant source **1207,** brine source **1206,** and base source **1208** can contain solutions having concentrations over the concentration of the components to be used in recharging the reusable modules. Water source **1205** can dilute the disinfectant, brine, and base from the fluid sources prior to recharging. In FIG. 12B, zirconium phosphate pump **1210** can pump disinfectant into the zirconium phosphate module **1203** with in-line mixing of concentrated disinfectant from disinfectant source **1207** from valve **1212** through junctions **1260** and **1261** and into static mixer **1218.** Concurrently, zirconium phosphate pump **1209** can pump water through junction **1259** and valve **1213** and into static mixer **1218** from water source **1205.** Alternatively, the concentrated disinfectant and water can be mixed through fluid line mixing at the junction of the two fluid lines. The zirconium phosphate pumps **1209** and **1210** can pump a disinfectant solution having a specified concentration and composition to disinfect the zirconium phosphate module **1203** via valves **1212** and **1213.** The disinfectant solution can flow from static mixer **1218** through valve **1214** to valve **1216** and then into the zirconium phosphate module **1203** through zirconium phosphate module inlet **1226.** Fluid can exit zirconium phosphate module **1203** through zirconium phosphate module outlet **1227** into zirconium phosphate effluent line **1230.** After disinfection of zirconium oxide module **1203,** zirconium phosphate pumps **1209 1210** can pump water from water source **1205** into zirconium phosphate module **1203.** For example, zirconium phosphate pump **1209** can pump water through valve **1213** to zirconium phosphate module **1203** while zirconium phosphate pump **1210** can pump water through valves **1211** and **1212** to zirconium phosphate module **1203.** Alternatively, zirconium phosphate pump **1209** can pump water through valves **1211, 1212,** and **1213** while zirconium phosphate pump **1210** pumps water through valves **1211** and **1212.** During recharging, zirconium phosphate pumps **1209** and **1210** can pump brine through valve **1211** to valve **1212** from brine source **1206** into static mixer **1218.** If a concentrated brine solution is being used, zirconium phosphate pumps **1209** and/or **1210** can pump water from water source **1205** to static mixer **1218** to dilute the brine solution and generate a brine solution having a proper solute concentration for recharging the zirconium phosphate. After pumping brine through the zirconium phosphate module **1203,** zirconium phosphate pump **1209** can pump water through valves **1211, 1212** and **1213** while zirconium phosphate pump **1210** can pump water through valve **1211** and **1212.**

The zirconium phosphate recharging flow path **1201** of FIG. 12B can have a heater **1224** and heat exchanger **1225.** One or more heat exchangers and one or more heaters can be used. The brine solution can be heated by the heater **1224** upstream of the zirconium phosphate module **1203.** Heat exchanger **1225** can utilize the heat from brine exiting the zirconium phosphate module **1203,** to heat the incoming brine solution upstream of heater **1224** to reduce the burden on heater **1224.** As described, the zirconium phosphate recharging flow path **1201** can also have an optional zirconium phosphate module bypass line **1228** fluidly connecting valve **1215** in the zirconium phosphate inlet line to valve **1217** in the zirconium phosphate effluent line **1230.** The zirconium phosphate module bypass line **1228** can neutralize the zirconium oxide effluent with brine even if the zirconium phosphate module **1203** is not being recharged. Zirconium phosphate recharging flow path **1201** can have a rinse loop **1229** connecting valve **1214** upstream of the heater **1224** and heat exchanger **1225** to valve **1216** to bypass heater **1224** and heat exchanger **1225** to rinse brine out of the zirconium phosphate module **1203.**

Various sensors can be included in the zirconium phosphate recharging flow path **1201** to ensure fluid parameters are within acceptable ranges. In FIG. 12B, conductivity sensor **1219** can be placed downstream of static mixer **1218** to ensure mixing and specified recharging fluid concentrations. Pressure sensor **1220** can measure the fluid pressure and to identify leaks or occlusions. Flow sensor **1222** can determine the flow rate of the fluid entering the zirconium phosphate module **1203** and control zirconium phosphate pumps **1209** and **1210.** Temperature sensor **1221** can determine if the recharging fluid is a proper temperature range upon entering zirconium phosphate module **1203** and relay data to a processor (not shown) that can control heater **1224.** Temperature sensor **1223** can determine the temperature of the zirconium phosphate effluent prior to entering heat exchanger **1225.** Other sensor arrangements, including any number of conductivity, pressure, flow, and temperature sensors can be used.

In FIG. 12C, zirconium oxide pump **1232** can pump disinfectant from disinfectant source **1207** through valve **1234** and into static mixer **1238** to disinfect the zirconium oxide module **1204** in zirconium oxide recharging flow path **1202.** Zirconium oxide pump **1231** can pump water from water source **1205** through valve **1235** to static mixer **1238** to dilute the disinfectant from disinfectant source **1207** to provide in-line mixing of the disinfectant solution. The diluted disinfectant can then be pumped through valve **1236** to zirconium oxide module inlet **1243** and into zirconium oxide module **1204.** Effluent from the zirconium oxide module **1204** can exit through zirconium oxide module outlet **1244** and into zirconium oxide effluent line **1245.** After disinfection, the disinfectant can be rinsed from the zirconium oxide module **1204** by pumping water from water source **1205** through valve **1235** to zirconium oxide module **1204** by zirconium oxide pump **1231** while zirconium oxide pump **1232** pumps water through valves **1233** and **1234** to zirconium oxide module **1204.** Alternatively, zirconium oxide pump **1231** can pump water through valves **1233, 1234,** and **1231,** while zirconium oxide pump **1232** pumps water through valves **1233** and **1234.** To recharge zirconium oxide module **1204,** zirconium oxide pump **1232** can pump base from base source **1208** through valves **1233** and **1234** through junctions **1264** and **1265** to static mixer **1238.** Water from water source **1205** can be pumped by zirconium oxide pump **1231** through junctions **1263** and **1265** into static mixer **1238** to dilute the base by in-line mixing. Alternatively, the water and base can be mixed through fluid line mixing at the junction of the two fluid lines. Alternatively, the base can be pre-set using specified amounts of base in pre-packaged packets or containers. Diluted base can flow through the zirconium oxide recharging flow path **1202** and through zirconium oxide module **1204.** The zirconium oxide module **1204** can be rinsed any numbers of times, as needed, by introducing water from water source **1205** to the zirconium oxide module **1204.** The zirconium oxide recharging flow path **1202** can also have a zirconium oxide module bypass line **1242** that fluidly connects valve **1236** to valve **1237** in the zirconium oxide effluent line **1245** to bypass zirconium oxide module **1204.** In this way, zirconium phosphate effluent can be neutralized with a base solution even if the zirconium oxide module **1204** is not being recharged. A heater and heat exchanger (not shown) can be positioned in the zirconium oxide recharging flow path **1202** to heat fluids prior to entering zirconium oxide module **1204.** A zirconium oxide rinse loop (not shown) can also be included to bypass the heater and heat exchanger. Similarly, the zirconium oxide recharging flow path **1202** can also have sensors for measurement and control over the recharging process. In FIG. 12C, a conductivity sensor **1239** can be placed downstream of static mixer **1238** to ensure that diluted recharging solutions have a desired concentration. Pressure sensor **1240** can detect the pressure in the zirconium oxide recharging flow path **1202** to detect leaks or occlusions. Flow sensor **1241** can detect the flow rate of fluid in the zirconium oxide recharging flow path **1202** and can to control zirconium oxide pumps **1231** and **1232.**

As shown in FIG. 12A, the present invention can provide in-line neutralization of the effluent from each of the zirconium phosphate recharging flow path **1201** and zirconium oxide recharging flow path **1202.** The zirconium phosphate effluent line **1230** can be fluidly connected to zirconium oxide effluent line **1245** at effluent line junction **1246** and fluidly connected to drain line **1247.** As shown in FIG.'s 12B and 12C, a static mixer **1248** can be positioned at or downstream of the effluent line junction **1246** to ensure mixing of the effluents from the zirconium phosphate recharging flow path **1201** and zirconium oxide recharging flow path **1202.** The combined effluent can be passed through the drain line **1247** to drain **1253,** a common waste reservoir (not shown), or separate waste reservoirs. A conductivity sensor **1250** as shown in FIG. 12B in zirconium phosphate effluent line **1230** and a conductivity sensor **1252** as shown in FIG. 12C in zirconium oxide effluent line **1245** can determine the composition of the effluents. Flow sensor **1249** in zirconium phosphate effluent line **1230** of FIG. 12B and flow sensor **1251** in zirconium oxide effluent line **1245** of FIG. 12C can be used simultaneously or independently to measure the flow rates of each of the effluents. Determining the composition of the effluent fluids as well as the respective flow rates using one or more sensors described can monitor the system function and ensure he combined effluent in drain line **1247** is safe for disposal or storage.

Brine source **1206,** disinfectant source **1207,** and base source **1208** can have filter **1254,** filter **1255,** and filter **1256,** respectively to remove particulate matter prior to entering zirconium phosphate recharging flow path **1201** or zirconium oxide recharging flow path **1202.** The filters can also act as inline mixers to mix the solutions. Water source **1205** can have microbial filter **1262** to remove microbes from the water source **1205.** Brine source **1206,** disinfectant source **1207,** and base source **1208** can be housed outside of a recharger housing denoted by line **1257.** The brine solution, disinfectant solution, and base solution can be generated through in-line mixing as described. Alternatively, pre-mixed solutions, concentrates, or infusates can be introduced into brine source **1206,** disinfectant source **1207,** and base source **1208** and delivered to zirconium phosphate recharging flow path **1201** or zirconium oxide recharging flow path **1202.** For example, the brine solution in brine source **1206** can be pre-mixed or provide in pre-packaged amounts in the proper concentrations and introduced into brine source **1206,** disinfectant source **1207,** and base source **1208.**

In-line mixing can provide higher concentrations of solutes, lower fluid volumes required by the system, and physically smaller fluid reservoirs. The fluids should have suitable concentrations for use in the zirconium phosphate recharging flow path **1201** or zirconium oxide recharging flow path **1202.** For example, an initially high source of disinfectant, such as peracetic acid, can be used in a concentration of between 20% and 40%. The zirconium phosphate recharging flow path **1201** of FIG. 12B can dilute the peracetic acid source by a factor of 20:1 to 40:1 to generate a disinfectant recharging solution having a concentration between 0.5 % and 2%. In one embodiment the initial disinfectant concentration can be 32%. The initial disinfectant concentration can be any concentration greater than 1%. Similarly, the base solution can be sodium hydroxide having an initial concentration between 14M and 22M. The zirconium oxide recharging flow path **1202** of FIG. 12C can dilute the base solution by 18:1 to 22:1 to generate a base solution having a concentration between 0.8 and 1.0 M. In one embodiment the initial base concentration can be 6M. The initial base solution concentration can be any concentration greater than or equal to 0.5 M. The brine solution can also be diluted in-line to generate a brine solution having a proper recharging concentration. The brine source **1206** of FIG. 12A can be one or more reservoirs. For example, an acetic acid source, a sodium acetate source and a sodium chloride source can each be connected in place of single brine source **1206.** Alternatively, an acetic acid source, a base source, and a sodium chloride source can be connected in place of the single brine source **1206** with mixing of the base and acetic acid to generate the sodium acetate. The individual components can be added to the zirconium phosphate recharging flow path **1201** in the proper ratios to generate the recharging brine.

The chemicals used in the recharging process can be packaged and shipped in any form. The chemicals can be packaged and shipped as solutions, either in proper concentrations for use in recharging or with higher concentrations for use in inline mixing. In any embodiment, the chemicals may be packaged and shipped in pure form, such as 100% acetic acid or solid sodium chloride, sodium acetate, or sodium hydroxide.

FIG. 13 illustrates a non-limiting example of a timeline that can be used for concurrent or separate recharging of zirconium phosphate and zirconium oxide. The steps illustrated in FIG. 13 are generalized times for the specific actions illustrated in FIG.'s 1-10. Timeline **1301** shows recharging zirconium phosphate and timeline **1302** shows recharging zirconium oxide. As illustrated in timeline **1301,** the zirconium phosphate recharging process can begin by introducing a disinfectant, such as peracetic acid, into the zirconium phosphate module, shown as step **1303.** The time necessary to fill the zirconium phosphate module with the disinfectant can depend on the flow rate of the disinfectant solution and the volume of the zirconium phosphate module. The disinfectant can be delivered to the zirconium phosphate module in step **1303** at a flow rate of between 100 and 500 mL/min, which can fill a zirconium phosphate module in a time of between 5-10 minutes. Longer or shorter flushing times can be used depending on the need. After filling the zirconium phosphate with the disinfectant solution, the disinfectant solution can be held in the zirconium phosphate module to ensure disinfecting of the zirconium phosphate module in step **1304.** In any embodiment, the disinfectant can be held in the zirconium phosphate module for any length of time sufficient to disinfect the zirconium phosphate module, including between 5 and 20 minutes. The temperature of the disinfectant can be determined with a temperature sensor, and the hold time adjusted as necessary. For example, if the disinfectant temperature is 22°C, the hold time can be 5 minutes. The disinfectant can also be heated to minimize the necessary hold time by heating the disinfectant to room temperature if necessary. During the hold time, the disinfectant flow can be stopped or reduced to a low flow condition, such as 5 to 75 ml/min. Holding the disinfectant in the module can build up pressure in the module, requiring periodic venting. To maintain the volume after venting, during which some fluid may leak, the disinfectant can be pumped into the module at a low flow rate during the venting. Alternatively, during the hold time, the disinfectant flow rate can be set to between 5 and 75 ml/min to prevent pressure buildup while maintaining fluid volume in the modules. The disinfectant solution can then be flushed from the zirconium phosphate module in step **1305** by pumping water through the zirconium phosphate module. The water can flow through the zirconium phosphate module at a specified rate. A higher flow rate of the water in step **1305** will cause a quicker flush time. The water can be pumped through the zirconium phosphate module at a rate of between 300 and 500 mL/min. Depending on the size of the zirconium phosphate module, the zirconium phosphate module can be flushed in about 5-10 minutes. As described, the system can utilize one or more sensors, such as pH sensors or conductivity sensors in the zirconium phosphate effluent lines to determine if disinfectant is fully flushed in step **1305**. After flushing the disinfectant from the zirconium phosphate module in step **1305,** brine solution can be pumped through the zirconium phosphate module to recharge the zirconium phosphate module starting in step **1306**. The brine solution can be pumped through the zirconium phosphate module in step **1306** at any rate. One of skill in the art will understand that a higher flow rate of brine solution may decrease the time necessary to recharge the zirconium phosphate, but may also decrease the efficiency of the process, resulting in the need for additional brine. Conductivity or pH sensors can determine if the zirconium phosphate module has been fully filled with brine.

The brine flow rate can be set to any flow rate, including between 150 and 250 mL/min. Depending on the size of the zirconium phosphate module, between 5 and 10 minutes may be needed for brine to reach the sensors in the zirconium phosphate effluent line. Once brine has reached the sensors in the effluent line, the brine can flow through the zirconium phosphate module in step **1307** until recharging is complete. Recharging time can vary based on the flow rate of the brine solution, the concentration of the brine solution, and the temperature of the brine solution. For example, the brine solution can be heated during the recharging process between 65°C and 95°C. Recharging of zirconium phosphate can be more efficient at elevated temperatures. Conductivity sensors can determine if step **1308** has been completed by detecting the conducting of the fluid in the zirconium phosphate effluent line. If the conductivity of the effluent matches the conductivity of the brine, then no additional ions from the brine are being exchanged onto the zirconium phosphate, and recharging is complete. For example, steps **1308, 1309,** and **1310** represent brine solution being flushed from the zirconium phosphate module with water. Flushing can continue through step **1310** until the conductivity sensors in the zirconium phosphate effluent line determine no additional brine is being removed from the zirconium phosphate module.

As depicted in timeline **1302,** zirconium oxide can be recharged concurrently or independently of zirconium phosphate. In step **1311,** zirconium oxide recharging begins by rinsing the zirconium oxide module with water. The water rinse can flush leftover dialysate bicarbonate or any sodium hydroxide from the flow loop, which react violently with acid necessary for disinfection. After flushing the zirconium oxide module with water in step **1311,** disinfectant solution can be delivered to disinfect the module in step **1312.** The time necessary to fill the zirconium oxide module with disinfectant depends on the size of the zirconium oxide module and the flow rate of the disinfectant. Because less zirconium oxide is needed for dialysis than zirconium phosphate, the zirconium oxide module may be smaller than the zirconium phosphate module, and therefore fill faster in step **1312** as compared to the zirconium phosphate module in step **1303.** Upon filling, the disinfectant can be sequestered in the zirconium oxide module to allow for disinfection in step **1313.** The disinfectant can be held in the zirconium oxide module for any length of time, including between 5 and 20 minutes. The temperature of the disinfectant can be determined with a temperature sensor, and a hold time adjusted as necessary. For example, if the disinfectant temperature is 22°C, the hold time can be 5 minutes. The disinfectant can also be heated to minimize the necessary hold time. Upon disinfection, the disinfectant can be flushed from the zirconium oxide module in step **1314**.

In step **1315** the base solution flows through the zirconium oxide module to recharge the zirconium oxide. Step **1315** continues until a basic solution is detected in the zirconium oxide effluent line. During simultaneous recharging, the basic effluent from the zirconium oxide recharging flow path neutralizes the acidic effluent from the zirconium phosphate recharging flow path. Once a basic effluent is detected in step **1315**, the zirconium oxide recharging process can be halted until the acid brine is detected in the effluent of the zirconium phosphate module in step **1306**, which may occur later due to size differences of the zirconium phosphate and zirconium oxide modules. After the acidic effluent is detected in the zirconium phosphate module, shown as step **1306**, the base can continue to flow through the zirconium oxide module in step **1316**. The flow rate of the base solution in step 1316 can be any suitable rate. For example, the flow rate of the base solution can be between 30 and 150 mL/min. To ensure neutralization, the flow rate of the base in step **1316** can depend on the flow rate of the brine in step **1307**. A neutralization ratio can be calculated based on the relative pH, buffer capacity and concentration of the zirconium phosphate effluent and zirconium oxide effluent. For example, a neutralization ratio of 1.5:1 means that 1.5 liters of the zirconium phosphate effluent will be required to fully neutralize one liter of zirconium oxide effluent. The flow rate of the base in step **1316** can be set to half the flow rate of the brine solution, allowing full neutralization of both solutions. For example, the flow rate of the base in step **1316** can be between 75 and 125 mL/min if the neutralization ratio is 1.5:1 and the brine flow rate is between 150 and 250 mL/min.

After the brine solution is detected in the effluent of the zirconium phosphate and the flushing of the brine begins in step **1308**, the base solution can pass through the zirconium oxide module, shown as step **1317** until the brine is mostly or fully flushed from the zirconium phosphate module, shown as step **1309.** At this point, the base solution can be flushed from the zirconium oxide module, shown as step **1318.** After confirming that the base has been flushed from the zirconium oxide module, flushing is completed in step **1319.**

One of skill in the art will understand that the times and flow rates described in FIG. 13 can be altered within the scope of the invention. Higher flow rates can cause faster recharging of the modules. Times can be decreased by using more concentrated solutions, but may decrease efficiency. Specified concentrations, flow rates, and times can be set per the needs of the user, taking into account the cost of chemicals and need for fast recharging. The times and flow rates shown in zirconium oxide recharging timeline **1302** can also be altered to reduce idle time. For example, the flow rate of the base solution in step **1315** can be slowed down to reduce the time gap between steps **1315** and **1316.** If a single sorbent module is being recharged independently, or if a common waste reservoir either inside or outside of the recharger is used for the zirconium phosphate and zirconium oxide recharging flow paths, the times and flow rates shown in FIG. 13 can be adjusted. Synchronizing the zirconium phosphate timeline **1301** with the zirconium oxide timeline **1302** is unnecessary because effluent is no longer neutralized in-line.

The zirconium oxide and zirconium phosphate sorbent modules can be recharged and reused any number of times. Alternatively, the sorbent modules may have a defined useful life, including a maximum number of recharge and reuse cycles. When a sorbent module reaches the end of the sorbent module's useful life, the sorbent module can be recycled or disposed of. A disinfection only cycle can disinfect the sorbent modules for safe disposal and/or recycling at the end of the sorbent module's useful life. In a disinfection only cycle, the disinfectant can be pumped into the sorbent module as described but the other recharge solutions would not be used. After disinfection, and optionally rinsing of the sorbent module, the sorbent module can be disposed or recycled safely.

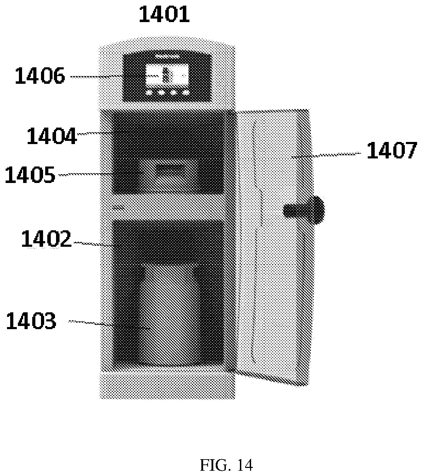

The rechargers described can be configured as shown in FIG. 14. The recharger **1401** includes a receiving compartment **1402** for receiving a reusable zirconium phosphate module **1403.** Fluid connections (not shown in FIG. 14) connect to the top and bottom of the zirconium phosphate module **1403** for passing recharging fluids into, through, and out of the reusable sorbent module **1403.** The recharging fluids replace ions bound to the sorbent materials during dialysis with new ions, recharging the zirconium phosphate within the zirconium phosphate module **1403,** allowing reuse of the zirconium phosphate module **1403** in dialysis. The recharger **1401** also has a second receiving compartment **1404** for receiving a reusable zirconium oxide module **1405,** which is also fluidly connected to recharging fluid sources for recharging of the zirconium oxide module **1405.** The recharger **1401** can be configured to concurrently recharge a zirconium phosphate module **1403** and a zirconium oxide module **1405,** or to independently recharge either a zirconium phosphate module **1403** or a zirconium oxide module **1405.** A user interface **1406** is provided to start or control the recharging process by the user. The user interface **1406** also provides the status of the recharging process to the user, such as the times of completion of each recharging step, or a time until the recharging process is complete. User interface **1406** also provides alert messages if any problems are detected during recharging, such as leaks, occlusions, pump failures, or mismatched chemicals. A door **1407** on the recharger **1401** controls access to the receiving compartments **1402** and **1404** during operation.

As shown in FIG. 14, the receiving compartments **1402** and **1404** may be of different sizes. Because less zirconium oxide is needed for dialysis than zirconium phosphate, the zirconium oxide module **1405** is smaller than the zirconium phosphate module **1403** and the receiving compartments **1402** and **1404** are sized accordingly. The zirconium phosphate receiving compartment **1402** is larger than the zirconium phosphate module **1403** and the zirconium oxide receiving compartment **1404** is larger than the zirconium oxide module **1405.** The larger space allows a user room to maneuver the fluid connectors and sorbent modules to connect the inlets and outlets on the sorbent modules to the inlets and outlets on the recharger. Rechargers with any number of receiving compartments for recharging any number or combination of zirconium oxide and/or zirconium phosphate sorbent modules can be constructed. For example, a recharger with two zirconium phosphate receiving compartments and two zirconium oxide receiving compartments can be similarly constructed. The rechargers can have 1, 2, 3, 4, 5, 6, or more receiving compartments, each capable of receiving zirconium oxide or zirconium phosphate sorbent modules.

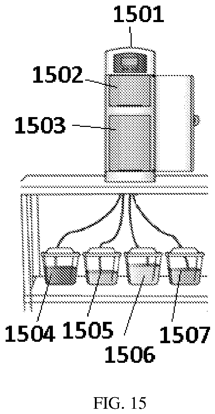

FIG. 15 illustrates non-limiting embodiment of a recharger set up for recharging zirconium oxide and zirconium phosphate, either concurrently or independently. To recharge the sorbent materials, one or more recharging fluids are passed through the reusable sorbent modules. As shown in FIG. 15, the recharger **1501** is fluidly connected to one or more recharging fluid sources, such as water source **1504,** brine source **1505,** disinfectant source **1506,** and base source **1507.** The recharger has a zirconium phosphate receiving compartment **1503** and a zirconium oxide receiving compartment **1502.** The recharger also has one or more pumps and valves (not shown in FIG. 15) for selectively delivering the recharging fluids from the fluid sources to the reusable modules. As shown in FIG. 15, the recharging fluid sources are housed external to the recharger **1501.** Alternatively the recharging fluid sources can be housed within the recharger **1501.** A drain line (not shown) is also connected to the recharger **1501** for disposal of waste fluids exiting the reusable modules. The drain line is fluidly connected to a drain, or alternatively, the drain line can be fluidly connected to one or more waste reservoirs for storage and later disposal.

The rechargers can be used in any setting, including a clinic, at home, or in a mobile setting. In any setting, the rechargers can use a water tank or any other source of potable or deionized water. For use in a mobile setting, vans or trucks can carry the rechargers, the disinfectant source, the brine solution, the base solution, and optionally the water, to a location for recharging. For at home use, the brine solution, disinfectant solution, base solution, and optionally the water, may be prepackaged and shipped to a patient. The patient can connect each of the sources to the recharger to allow recharging and reuse of the sorbent modules in dialysis. As described, the rechargers can provide for inline mixing of chemicals, reducing the amount of chemicals required to be moved for use in a mobile setting. Inline mixing of chemicals allows for a smaller amount of concentrated solutions to be moved to a location in a mobile or at home setting, and water from a local water source, such as municipal drinking water, can dilute the disinfectant, base, and/or brine inline. Alternatively, a deionized or purified water source can be provided in a mobile setting. Effluent from the sorbent modules can be collected and neutralized inline for immediate disposal in any drain, or can be collected for later neutralization and disposal offline. The ability to neutralize and dispose of the combined effluents in a drain allow for easier use in an at home or mobile setting, without the need for large waste reservoirs and further treatment.

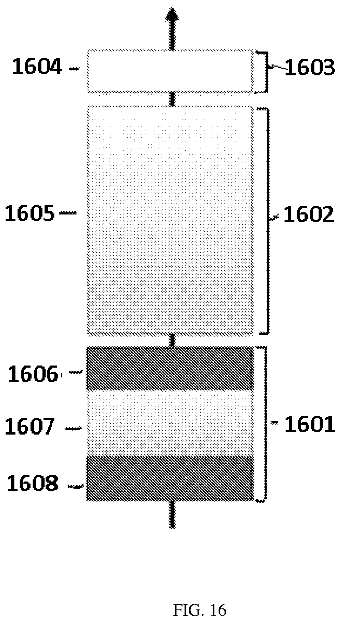

A non-limiting embodiment of a reusable sorbent cartridge having modules that can be separated and recharged by systems and methods of the present invention is shown in FIG. 16. The sorbent cartridge can be separated into reusable modules to facilitate recharging of one or more sorbent materials. In FIG. 16, the sorbent cartridge has a first sorbent module **1601,** a second sorbent module **1602,** and a third sorbent module **1603.** The first module **1601** can have a layer of activated carbon **1608,** a layer of alumina and urease **1607,** and a second layer of activated carbon **1606.** The activated carbon can remove many non-ionic solutes from the dialysate. The urease catalyzes the conversion of urea in the dialysate into ammonium ions. The alumina can serve as a support for the urease. The second layer of activated carbon **1606** can capture any urease that migrates out of alumina and urease layer **1607** prior to exiting the first module **1601.** The first module **1601** can be a single use module, or can be a multiple use module with replenishment of the urease. The second module **1602** can have zirconium phosphate **1605.** After dialysis, zirconium phosphate **1605** will contain bound potassium, calcium, magnesium, and ammonium ions, which can be replaced with sodium and hydrogen ions by the recharging process described herein. Third module **1603** can contain zirconium oxide **1604.** After use, the zirconium oxide **1604** will contain bound phosphate, fluoride and other anions, which can be replaced with hydroxide anions through the recharging process described herein. The flow direction of flow of dialysate through the sorbent cartridge is shown by the arrow in FIG. 16. The recharging solutions can also flow through the reusable sorbent modules in an opposite direction to improve the efficiency of the recharging process.

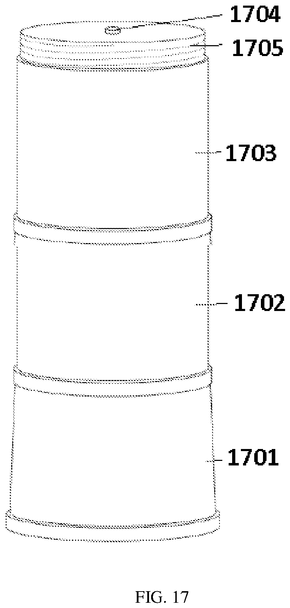

FIG. 17 illustrates another non-limiting example of a modular sorbent cartridge that can be used in the recharging process described herein. The modular sorbent cartridge can be separated into discrete modules including a first module **1701,** a second module **1702,** and a third module **1703** connected together to form a sorbent cartridge. The first module **1701** can contain activated carbon, urease, and alumina; the second module **1702** can contain zirconium phosphate; and the third module **1703** can contain zirconium oxide. One of skill in the art will understand that the modular sorbent cartridge illustrated in FIG. 17 is for illustrative purposes only, and modifications to the sorbent cartridge can be made within the scope of the invention. Alternatively, the sorbent modules can be independent with fluid lines connecting each of the sorbent modules for dialysis. During dialysis, dialysate can enter the sorbent cartridge through the bottom of first module **1701,** travel through modules **1701, 1702,** and **1703,** and exit through fluid outlet **1704.** The fluid outlet **1704** can connect to the rest of the dialysate flow path. Threaded portion **1705** on module **1703** can connect modules to each other, to the dialysate flow path, or to the recharger as described herein. The threaded portion **1705** can be included on any of the sorbent modules. Other connection types suitable for secured fluid connection in dialysis known in the art is contemplated by the invention. For example, fluid lines can be clamped directly onto fluid outlet **1704**. After dialysis, a user can disconnect the sorbent modules for disposal of single use modules and for recharging of the reusable modules.

One skilled in the art will understand that various combinations and/or modifications and variations can be made in the described systems and methods depending upon the specific needs for operation.

## Claims

1. A sorbent recharger (1401), comprising:
a first receiving compartment (1402) for a first sorbent module (1403); the receiving compartment (1402) having a first sorbent module inlet and a first sorbent module outlet;
a first inlet line fluidly connected to the first sorbent module inlet;
a first effluent line fluidly connected to the first sorbent module outlet;
at least one of an disinfectant source, a brine source, a base source, and a water source fluidly connected to the first inlet line;
at least a first pump positioned in the first inlet line for pumping fluid from the disinfectant source, brine source, and water source to the first sorbent module inlet;
at least one flow sensor, at least one pressure sensor, at least one temperature sensor, and at least one conductivity sensor; and
a control system in communication with at least one of the flow sensor, pressure sensor, temperature sensor or conductivity sensor; the control system for controlling the first pump, preferably said sorbent recharger (1401) further comprising a user interface (1406) in communication with the control system, wherein the control system determines whether at least one of the pressure, flow rate, temperature, and conductivity are within predetermined ranges, wherein the control system generates an alert indicating a leak when the pressure is below the predetermined range; and wherein the control system generates an alert indicating an occlusion when the pressure is above the predetermined range.

2. The sorbent recharger (1401) of claim 1, wherein at least one conductivity sensor is located upstream of the first sorbent module inlet;
wherein the control system determines the flow rate and conductivity of a fluid upstream of the first sorbent module; and wherein
the control system generates an alert indicating a pump failure when the flow rate is below the predetermined range and the conductivity of a fluid upstream of the first sorbent module inlet is within a predetermined range; and
wherein the control system generates an alert indicating a chemical run-out when the control system determines the flow rate is below the predetermined range and that the conductivity of the fluid upstream of the first sorbent module inlet is below the predetermined range.

3. The sorbent recharger (1401) of any preceding claim, further comprising a heater in the first inlet line, the temperature sensor in communication with the control system; wherein the control system controls the heater based on data from the temperature sensor, preferably wherein the control system generates an alert if the temperature in the first inlet line does not reach a predetermined temperature in a predetermined amount of time, preferably further comprising a heat exchanger; the heat exchanger fluidly connected to the first inlet line and the first effluent line,
preferably further comprising a second temperature sensor in the first effluent line, wherein the control system generates an alert if the temperature in the first effluent line does not reach a predetermined temperature in a predetermined amount of time, optionally wherein the control system calculates an amount of brine necessary for recharging a sorbent module containing zirconium phosphate based, at least in part, on the temperature in the first effluent line.

4. The sorbent recharger (1401) of any preceding claim, further comprising a second receiving compartment (1404) for a second sorbent module (1405); the second receiving compartment (1404) having a second sorbent module inlet and a second sorbent module outlet; a second inlet line fluidly connected to the second sorbent module inlet;
a second effluent line fluidly connected to the second sorbent module outlet;
wherein the disinfectant source, the base source, and the water source are fluidly connected to the second inlet line;
at least a second pump positioned in the second inlet line for pumping fluid from the disinfectant source, base source, and water source to the second sorbent module inlet;
at least one flow sensor, at least one pressure sensor, at least one temperature sensor, and at least one conductivity sensor positioned in the second inlet line;
wherein the control system is in communication with at least one of the flow sensor, pressure sensor, temperature sensor, and conductivity sensor; the control system controlling the second pump, preferably wherein the at least one conductivity sensor is positioned in the first effluent line; wherein the control system controls the first pump, the second pump, or both pumps to pump fluid from the disinfectant source, brine source, and/or water source through the first sorbent module; and wherein the control system determines a conductivity of fluid in the first effluent line based on data from the conductivity sensor positioned in the first effluent line;
wherein at least one conductivity sensor is positioned in the second effluent line; wherein the control system controls the second pump the first pump, the second pump, or both pumps to pump fluid from the disinfectant source, base source, and/or water source through the second sorbent module; and wherein the control system determines a conductivity of fluid in the second effluent line based on data from the conductivity sensor positioned in the second effluent line.

5. The sorbent recharger (1401) of claim 4, wherein the second effluent line is fluidly connected to the first effluent line at a junction; and further comprising a static mixer at or downstream of the junction, preferably wherein the control system calculates a neutralization ratio based on the conductivity of the fluid in the first effluent line and the conductivity of the fluid in the second effluent line; and wherein the control system controls the second pump and the first pump based on data from the conductivity sensor in the first effluent line and the conductivity sensor in the second effluent line;
wherein the control system controls the first pump and second pump to generate a fluid with a pH within a predetermined pH range in the static mixer based on the neutralization ratio, preferably wherein the predetermined pH range is between 5 and 9.

6. The sorbent recharger (1401) of claim 4, wherein the control system controls the first pump to pump fluid with an acidic pH through the first inlet line and controls the second pump to pump fluid with a basic pH through the second inlet line concurrently.

7. The sorbent recharger (1401) of claim 2, wherein the control system determines the flow rate, pressure, and conductivity of a fluid upstream of the first sorbent module at preset times.

8. The sorbent recharger (1401) of claim 5, wherein the control system stops the second pump when the conductivity of the fluid in the second effluent line reaches a predetermined range, preferably wherein the control system stops the first pump when the conductivity of the fluid in the first effluent line reaches a predetermined range, preferably wherein the control system starts the first pump and second pump when the conductivity in the first effluent line reaches a predetermined range.

9. The sorbent recharger (1401) of any preceding claim, wherein the control system controls the first pump to pump water from the water source through the first inlet line after pumping a first fluid through the first inlet line and before pumping a second fluid through the first inlet line.

10. A method, comprising the steps of:
pumping fluid from an disinfectant source (1107), a base source (1108), a brine source (1106), a water source (1105), or combinations thereof, through a recharging flow path (1101) to a first sorbent module (1103); and
determining a presence of a leak, occlusion, pump failure, or chemical run-out, wherein determining the presence of a leak comprises determining that a pressure in the recharging flow path is below a predetermined range, wherein determining the presence of an occlusion comprises determining that a pressure in the recharging flow path (1101) is above a predetermined range, wherein determining the presence of a pump failure comprises the steps of:
determining that a flow rate in the recharging flow path (1101) is below a predetermined range; and
determining that a conductivity at a sorbent module inlet of the first sorbent module (1104) is within a predetermined range, wherein determining the presence of a chemical run out comprises the steps of determining that a flow rate in the recharging flow path (1101) is below a predetermined range; and
determining that a conductivity at a sorbent module inlet of the first sorbent module (1103) is below a predetermined range.

11. The method of claim 10, further comprising the steps of pumping fluid from an disinfectant source (1107), a base source (1108), a brine source (1106), a water source (1105), or combinations thereof through the recharging flow path (1101) to a second sorbent module (1104); and
pumping fluid through a first effluent line fluidly connected to the first sorbent module (1103) and a second effluent line fluidly connected to the second sorbent module (1104) to a static mixer (1146) or a common reservoir.

12. The method of claim 11, further comprising:
determining a conductivity of a fluid in the first effluent line and determining and conductivity of a fluid in the second effluent line; and
calculating a neutralization ratio based on a conductivity of fluid in the first effluent line and the second effluent line;
wherein the step of pumping fluid from the first effluent line and the second effluent line to the static mixer (1146) or common reservoir comprises controlling a flow rate of the fluid in the first effluent line and second effluent line based on the neutralization ratio to generate a fluid in the static mixer or common reservoir within a predetermined pH range.

13. The method of any of claims 10-12, further comprising the step of:
determining a temperature of the fluid wherein the fluid is a brine solution in a first effluent line fluidly connected to the first sorbent module (1103);
calculating an amount of the brine solution necessary for recharging zirconium phosphate wherein the first sorbent module (1103) contains zirconium phosphate based, at least in part, on the temperature in the first effluent line,
preferably further comprising the steps of:
pumping fluid from the disinfectant source (1107) into the first sorbent module (1103);
determining a conductivity in a first effluent line; and
stopping the step of pumping the fluid from the disinfectant source (1107) if the conductivity in the first effluent line is within a predetermined range, optionally further comprising the step of pumping water from the water source (1105) into the first sorbent module (1103) at a predetermined time after the step of stopping the pumping of fluid from the disinfectant source (1107), optionally further comprising the step of venting the first sorbent module (1103) at a predetermined time after stopping pumping of fluid from the disinfectant source (1107), optionally further comprising the step of pumping fluid from the disinfectant source (1107) into the first sorbent module (1103) while venting the first sorbent module (1103).

14. The method of any of claims 10 to 13, wherein the first sorbent module (1103) contains zirconium oxide, and the method further comprises the step of determining an amount of base pumped through the first sorbent module (1103).

## Patentansprüche

1. Sorptionsmittelwiederauffüllvorrichtung (1401), umfassend:
eine erste Aufnahmekammer (1402) für ein erstes Sorptionsmittelmodul (1403); wobei die Aufnahmekammer (1402) einen ersten Sorptionsmittelmoduleinlass und einen ersten Sorptionsmittelmodulauslass aufweist;
eine erste Einlassleitung, die mit dem ersten Sorptionsmittelmoduleinlass in Fluidverbindung steht;
eine erste Abflussleitung, die mit dem ersten Sorptionsmittelmodulauslass in Fluidverbindung steht;
eine Desinfektionsmittelquelle, eine Solequelle, eine Basisquelle und/oder eine Wasserquelle, die mit der ersten Einlassleitung in Fluidverbindung steht;
mindestens eine erste Pumpe, die in der ersten Einlassleitung positioniert ist, um Fluid von der Desinfektionsmittelquelle, der Solequelle und der Wasserquelle zum ersten Sorptionsmittelmoduleinlass zu pumpen;
mindestens einen Durchflusssensor, mindestens einen Drucksensor, mindestens einen Temperatursensor und mindestens einen Leitfähigkeitssensor; und
ein Steuersystem, das mit dem Durchflusssensor, dem Drucksensor, dem Temperatursensor und/oder dem Leitfähigkeitssensor kommuniziert; wobei das Steuersystem zum Steuern der ersten Pumpe, vorzugsweise der Sorptionsmittelwiederauffüllvorrichtung (1401), ferner eine Benutzerschnittstelle (1406) umfasst, die mit dem Steuersystem kommuniziert, wobei das Steuersystem bestimmt, ob der Druck, die Durchflussrate, die Temperatur und/oder die Leitfähigkeit innerhalb vorbestimmter Bereiche liegen, wobei das Steuersystem eine Warnmeldung erzeugt, die ein Leck anzeigt, wenn der Druck unterhalb des vorbestimmten Bereichs liegt; und wobei das Steuersystem eine Warnmeldung erzeugt, die eine Verstopfung anzeigt, wenn der Druck oberhalb des vorbestimmten Bereichs liegt.

2. Sorptionsmittelwiederauffüllvorrichtung (1401) nach Anspruch 1, wobei mindestens ein Leitfähigkeitssensor stromaufwärts des ersten Sorptionsmittelmoduleinlasses angeordnet ist;
wobei das Steuersystem die Durchflussrate und die Leitfähigkeit eines Fluids stromaufwärts des ersten Sorptionsmittelmoduls bestimmt; und wobei
das Steuersystem eine Warnmeldung erzeugt, die einen Pumpenausfall anzeigt, wenn die Durchflussrate unter dem vorbestimmten Bereich liegt und die Leitfähigkeit eines Fluids stromaufwärts des ersten Sorptionsmittelmoduleinlasses innerhalb eines vorbestimmten Bereichs liegt; und
wobei das Steuersystem eine Warnmeldung erzeugt, die ein chemisches Auslaufen anzeigt, wenn das Steuersystem bestimmt, dass die Durchflussrate unter dem vorgegebenen Bereich liegt und dass die Leitfähigkeit des Fluids stromaufwärts des ersten Sorptionsmittelmoduleinlasses unter dem vorgegebenen Bereich liegt.

3. Sorptionsmittelwiederauffüllvorrichtung (1401) nach einem der vorhergehenden Ansprüche, ferner umfassend eine Heizung in der ersten Einlassleitung, wobei der Temperatursensor mit dem Steuersystem kommuniziert; wobei das Steuersystem die Heizung auf der Grundlage von Daten von dem Temperatursensor steuert, wobei vorzugsweise das Steuersystem eine Warnmeldung erzeugt, wenn die Temperatur in der ersten Einlassleitung eine vorbestimmte Temperatur in einer vorbestimmten Zeitspanne nicht erreicht, vorzugsweise ferner umfassend einen Wärmetauscher; wobei der Wärmetauscher in Fluidverbindung mit der ersten Einlassleitung und der ersten Abflussleitung steht,
vorzugsweise ferner umfassend einen zweiten Temperatursensor in der ersten Abflussleitung, wobei das Steuersystem eine Warnmeldung erzeugt, wenn die Temperatur in der ersten Abflussleitung eine vorbestimmte Temperatur in einer vorbestimmten Zeitspanne nicht erreicht, wobei das Steuersystem optional eine Solemenge berechnet, die zum Wiederauffüllen eines Sorptionsmittelmoduls erforderlich ist, das Zirkoniumphosphat enthält, zumindest teilweise basierend auf der Temperatur in der ersten Abflussleitung.

4. Sorptionsmittelwiederauffüllvorrichtung (1401) nach einem der vorhergehenden Ansprüche, ferner umfassend eine zweite Aufnahmekammer (1404) für ein zweites Sorptionsmittelmodul (1405); wobei die zweite Aufnahmekammer (1404) einen zweiten Sorptionsmittelmoduleinlass und einen zweiten Sorptionsmittelmodulauslass aufweist;
eine zweite Einlassleitung mit dem zweiten Sorptionsmittelmoduleinlass in Fluidverbindung steht;
eine zweite Abflussleitung mit dem zweiten Sorptionsmittelmodulauslass in Fluidverbindung steht;
wobei die Desinfektionsmittelquelle, die Basisquelle und die Wasserquelle mit der zweiten Einlassleitung in Fluidverbindung stehen;
mindestens eine zweite Pumpe, die in der zweiten Einlassleitung positioniert ist, um Fluid von der Desinfektionsmittelquelle, der Basisquelle und der Wasserquelle zum zweiten Sorptionsmittelmoduleinlass zu pumpen;
mindestens einen Durchflusssensor, mindestens einen Drucksensor, mindestens einen Temperatursensor und mindestens einen Leitfähigkeitssensor, die in der zweiten Einlassleitung positioniert sind;
wobei das Steuersystem mit dem Durchflusssensor, dem Drucksensor, dem Temperatursensor und/oder dem Leitfähigkeitssensor kommuniziert; wobei das Steuersystem die zweite Pumpe steuert, wobei vorzugsweise der mindestens eine Leitfähigkeitssensor in der ersten Abflussleitung positioniert ist; wobei das Steuersystem die erste Pumpe, die zweite Pumpe oder beide Pumpen steuert, um Fluid von der Desinfektionsmittelquelle, der Solequelle und/oder der Wasserquelle durch das erste Sorptionsmittelmodul zu pumpen; und wobei das Steuersystem eine Leitfähigkeit des Fluids in der ersten Abflussleitung bestimmt, basierend auf Daten von dem in der ersten Abflussleitung positionierten Leitfähigkeitssensor;
wobei mindestens ein Leitfähigkeitssensor in der zweiten Abflussleitung positioniert ist; wobei das Steuersystem die zweite Pumpe, die erste Pumpe, die zweite Pumpe oder beide Pumpen steuert, um Fluid von der Desinfektionsmittelquelle, der Basisquelle und/oder der Wasserquelle durch das zweite Sorptionsmittelmodul zu pumpen; und wobei das Steuersystem eine Leitfähigkeit von Fluid in der zweiten Abflussleitung bestimmt, basierend auf Daten von dem in der zweiten Abflussleitung positionierten Leitfähigkeitssensor.

5. Sorptionsmittelwiederauffüllvorrichtung (1401) nach Anspruch 4, wobei die zweite Abflussleitung mit der ersten Abflussleitung an einer Verbindungsstelle in Fluidverbindung steht und ferner einen statischen Mischer an oder stromabwärts der Verbindungsstelle umfasst, wobei vorzugsweise das Steuersystem ein Neutralisationsverhältnis basierend auf der Leitfähigkeit des Fluids in der ersten Abflussleitung und der Leitfähigkeit des Fluids in der zweiten Abflussleitung berechnet; und wobei das Steuersystem die zweite Pumpe und die erste Pumpe basierend auf den Daten des Leitfähigkeitssensors in der ersten Abflussleitung und des Leitfähigkeitssensors in der zweiten Abflussleitung steuert;
wobei das Steuersystem die erste Pumpe und die zweite Pumpe steuert, um ein Fluid mit einem pH-Wert innerhalb eines vorbestimmten pH-Bereichs in dem statischen Mischer basierend auf dem Neutralisationsverhältnis zu erzeugen, wobei vorzugsweise der vorbestimmte pH-Bereich zwischen 5 und 9 liegt.

6. Sorptionsmittelwiederauffüllvorrichtung (1401) nach Anspruch 4, wobei das Steuersystem die erste Pumpe steuert, um Fluid mit einem sauren pH-Wert durch die erste Einlassleitung zu pumpen, und die zweite Pumpe steuert, um gleichzeitig Fluid mit einem basischen pH-Wert durch die zweite Einlassleitung zu pumpen.

7. Sorptionsmittelwiederauffüllvorrichtung (1401) nach Anspruch 2, wobei das Steuersystem die Durchflussrate, den Druck und die Leitfähigkeit eines Fluids stromaufwärts des ersten Sorptionsmittelmoduls zu voreingestellten Zeiten bestimmt.

8. Sorptionsmittelwiederauffüllvorrichtung (1401) nach Anspruch 5, wobei das Steuersystem die zweite Pumpe stoppt, wenn die Leitfähigkeit des Fluids in der zweiten Abflussleitung einen vorbestimmten Bereich erreicht, wobei vorzugsweise das Steuersystem die erste Pumpe stoppt, wenn die Leitfähigkeit des Fluids in der ersten Abflussleitung einen vorbestimmten Bereich erreicht, wobei vorzugsweise das Steuersystem die erste Pumpe und die zweite Pumpe startet, wenn die Leitfähigkeit in der ersten Abflussleitung einen vorbestimmten Bereich erreicht.

9. Sorptionsmittelwiederauffüllvorrichtung (1401) nach einem der vorhergehenden Ansprüche, wobei das Steuersystem die erste Pumpe steuert, um Wasser aus der Wasserquelle durch die erste Einlassleitung zu pumpen, nachdem ein erstes Fluid durch die erste Einlassleitung gepumpt wurde und bevor ein zweites Fluid durch die erste Einlassleitung gepumpt wird.

10. Verfahren, umfassend die folgenden Schritte:
Pumpen von Fluid von einer Desinfektionsmittelquelle (1107), einer Basisquelle (1108), einer Solequelle (1106), einer Wasserquelle (1105) oder Kombinationen davon durch einen Wiederauffülldurchflusskanal (1101) zu einem ersten Sorptionsmittelmodul (1103); und
Bestimmen des Vorhandenseins eines Lecks, einer Verstopfung, eines Pumpenausfalls oder eines chemischen Auslaufens, wobei das Bestimmen des Vorhandenseins eines Lecks das Bestimmen umfasst, dass ein Druck in dem Wiederauffülldurchflusskanal unterhalb eines vorbestimmten Bereichs liegt, wobei das Bestimmen des Vorhandenseins einer Verstopfung das Bestimmen umfasst, dass ein Druck in dem Wiederauffülldurchflusskanal (1101) oberhalb eines vorbestimmten Bereichs liegt, wobei das Bestimmen des Vorhandenseins eines Pumpenausfalls die folgenden Schritte umfasst:
Bestimmen, dass eine Durchflussrate im Wiederauffülldurchflusskanal (1101) unterhalb eines vorbestimmten Bereichs liegt; und
Bestimmen, dass eine Leitfähigkeit an einem Sorptionsmittelmoduleinlass des ersten Sorptionsmittelmoduls (1104) innerhalb eines vorbestimmten Bereichs liegt, wobei das Bestimmen des Vorhandenseins eines chemischen Auslaufens die Schritte umfasst: Bestimmen, dass eine Durchflussrate in dem Wiederauffülldurchflusskanal (1101) unterhalb eines vorbestimmten Bereichs liegt; und
Bestimmen, dass eine Leitfähigkeit an einem Sorptionsmittelmoduleinlass des ersten Sorptionsmittelmoduls (1103) unter einem vorbestimmten Bereich liegt.

11. Verfahren nach Anspruch 10, ferner umfassend die Schritte des Pumpens von Fluid von einer Desinfektionsmittelquelle (1107), einer Basisquelle (1108), einer Solequelle (1106), einer Wasserquelle (1105) oder Kombinationen davon durch den Wiederauffülldurchflusskanal (1101) zu einem zweiten Sorptionsmittelmodul (1104); und
Pumpen von Fluid durch eine erste Abflussleitung, die mit dem ersten Sorptionsmittelmodul (1103) in Fluidverbindung steht, und eine zweite Abflussleitung, die mit dem zweiten Sorptionsmittelmodul (1104) in Fluidverbindung steht, zu einem statischen Mischer (1146) oder einem gemeinsamen Reservoir.

12. Verfahren nach Anspruch 11, ferner umfassend:
Bestimmen einer Leitfähigkeit eines Fluids in der ersten Abflussleitung und Bestimmen einer Leitfähigkeit eines Fluids in der zweiten Abflussleitung; und
Berechnen eines Neutralisationsverhältnisses basierend auf der Leitfähigkeit des Fluids in der ersten Abflussleitung und in der zweiten Abflussleitung;
wobei der Schritt des Pumpens von Fluid aus der ersten Abflussleitung und der zweiten Abflussleitung zu dem statischen Mischer (1146) oder dem gemeinsamen Reservoir das Steuern einer Durchflussrate des Fluids in der ersten Abflussleitung und der zweiten Abflussleitung auf der Grundlage des Neutralisationsverhältnisses umfasst, um ein Fluid in dem statischen Mischer oder dem gemeinsamen Reservoir innerhalb eines vorbestimmten pH-Bereichs zu erzeugen.

13. Verfahren nach einem der Ansprüche 10-12, ferner umfassend die folgenden Schritte:
Bestimmen einer Temperatur des Fluids, wobei das Fluid eine Solelösung in einer ersten Abflussleitung ist, die mit dem ersten Sorptionsmittelmodul (1103) in Fluidverbindung steht;
Berechnen einer Menge der Solelösung, die zum Wiederauffüllen von Zirkoniumphosphat erforderlich ist, wobei das erste Sorptionsmittelmodul (1103) Zirkoniumphosphat enthält, zumindest teilweise basierend auf der Temperatur in der ersten Abflussleitung,
vorzugsweise ferner umfassend die folgenden Schritte:
Pumpen von Fluid aus der Desinfektionsmittelquelle (1107) in das erste Sorptionsmittelmodul (1103);
Bestimmen einer Leitfähigkeit in einer ersten Abflussleitung; und
Stoppen des Schritts des Pumpens des Fluids aus der Desinfektionsmittelquelle (1107), wenn die Leitfähigkeit in der ersten Abflussleitung innerhalb eines vorbestimmten Bereichs liegt, optional ferner umfassend den Schritt des Pumpens von Wasser aus der Wasserquelle (1105) in das erste Sorptionsmittelmodul (1103) zu einem vorbestimmten Zeitpunkt nach dem Schritt des Stoppens des Pumpens von Fluid aus der Desinfektionsmittelquelle (1107), optional ferner umfassend den Schritt des Entlüftens des ersten Sorptionsmittelmoduls (1103) zu einem vorbestimmten Zeitpunkt nach dem Stoppen des Pumpens von Fluid aus der Desinfektionsmittelquelle (1107), optional ferner umfassend den Schritt des Pumpens von Fluid aus der Desinfektionsmittelquelle (1107) in das erste Sorptionsmittelmodul (1103) während des Entlüftens des ersten Sorptionsmittelmoduls (1103).

14. Verfahren nach einem der Ansprüche 10 bis 13, wobei das erste Sorptionsmittelmodul (1103) Zirkoniumoxid enthält und das Verfahren ferner den Schritt des Bestimmens einer durch das erste Sorptionsmittelmodul (1103) gepumpten Basenmenge umfasst.

## Revendications

1. Dispositif de recharge de sorbant (1401), comprenant :
un premier compartiment de réception (1402) pour un premier module de sorbant (1403) ; le compartiment de réception (1402) ayant une entrée de premier module de sorbant et une sortie de premier module de sorbant ;
une première conduite d'entrée reliée fluidiquement à l'entrée de premier module de sorbant ;
une première conduite d'effluent reliée fluidiquement à la sortie de premier module de sorbant ;
une source de désinfectant, et/ou une source de saumure, et/ou une source de base et/ou une source d'eau reliées fluidiquement à la première conduite d'entrée ;
au moins une première pompe positionnée dans la première conduite d'entrée pour pomper du fluide depuis la source de désinfectant, la source de saumure et la source d'eau vers l'entrée de premier module de sorbant ;
au moins un capteur d'écoulement, au moins un capteur de pression, au moins un capteur de température et au moins un capteur de conductivité ; et
un système de commande en communication avec le capteur de débit, et/ou le capteur de pression, et/ou le capteur de température et/ou le capteur de conductivité ; le système de commande destiné à commander la première pompe, de préférence ledit dispositif de recharge de sorbant (1401) comprenant en outre une interface utilisateur (1406) en communication avec le système de commande, le système de commande déterminant si la pression, et/ou le taux d'écoulement, et/ou la température et/ou la conductivité sont à l'intérieur de plages prédéterminées, le système de commande générant une alerte indiquant une fuite lorsque la pression est inférieure à la plage prédéterminée ; et le système de commande générant une alerte indiquant une occlusion lorsque la pression est supérieure à la plage prédéterminée.

2. Dispositif de recharge de sorbant (1401) selon la revendication 1, dans lequel au moins un capteur de conductivité est situé en amont de l'entrée de premier module de sorbant ;
dans lequel le système de commande détermine le taux d'écoulement et la conductivité d'un fluide en amont du premier module de sorbant ; et dans lequel
le système de commande génère une alerte indiquant une défaillance de pompe lorsque le taux d'écoulement est inférieur à la plage prédéterminée et que la conductivité d'un fluide en amont de l'entrée de premier module de sorbant se trouve à l'intérieur d'une plage prédéterminée ; et
dans lequel le système de commande génère une alerte indiquant un manque de produit chimique lorsque le système de commande détermine que le taux d'écoulement est inférieur à la plage prédéterminée et que la conductivité du fluide en amont de l'entrée de premier module de sorbant est inférieure à la plage prédéterminée.

3. Dispositif de recharge de sorbant (1401) selon l'une quelconque des revendications précédentes, comprenant en outre un dispositif de chauffage dans la première conduite d'entrée, le capteur de température étant en communication avec le système de commande ; dans lequel le système de commande commande le dispositif de chauffage sur la base de données provenant du capteur de température, de préférence dans lequel le système de commande génère une alerte si la température dans la première conduite d'entrée n'atteint pas une température prédéterminée dans une durée prédéterminée, de préférence comprenant en outre un échangeur de chaleur ; l'échangeur de chaleur étant relié fluidiquement à la première conduite d'entrée et à la première conduite d'effluent,
de préférence comprenant en outre un second capteur de température dans la première conduite d'effluent, dans lequel le système de commande génère une alerte si la température dans la première conduite d'effluent n'atteint pas une température prédéterminée dans une durée prédéterminée, éventuellement dans lequel le système de commande calcule une quantité de saumure nécessaire à la recharge d'un module de sorbant contenant du phosphate de zirconium sur la base, au moins en partie, de la température dans la première conduite d'effluent.

4. Dispositif de recharge de sorbant (1401) selon l'une quelconque des revendications précédentes, comprenant en outre un second compartiment de réception (1404) pour un second module de sorbant (1405) ; le second compartiment de réception (1404) ayant une entrée de second module de sorbant et une sortie de second module de sorbant ;
une seconde conduite d'entrée reliée fluidiquement à l'entrée de second module de sorbant ;
une seconde conduite d'effluent reliée fluidiquement à la sortie de second module de sorbant ;
dans lequel la source de désinfectant, la source de base et la source d'eau sont reliées fluidiquement à la seconde conduite d'entrée ;
au moins une seconde pompe positionnée dans la seconde conduite d'entrée pour pomper du fluide depuis la source de désinfectant, la source de base et la source d'eau vers l'entrée de second module de sorbant ;
au moins un capteur d'écoulement, au moins un capteur de pression, au moins un capteur de température et au moins un capteur de conductivité positionné dans la seconde conduite d'entrée ;
dans lequel le système de commande est en communication avec le capteur d'écoulement, et/ou le capteur de pression, et/ou le capteur de température et/ou le capteur de conductivité ; le système de commande commandant la seconde pompe, de préférence dans lequel l'au moins un capteur de conductivité est positionné dans la première conduite d'effluent ; dans lequel le système de commande commande la première pompe, la seconde pompe ou les deux pompes pour pomper du fluide depuis la source de désinfectant, la source de saumure et/ou la source d'eau à travers le premier module de sorbant ; et dans lequel le système de commande détermine une conductivité de fluide dans la première conduite d'effluent sur la base de données provenant du capteur de conductivité positionné dans la première conduite d'effluent ;
dans lequel au moins un capteur de conductivité est positionné dans la seconde conduite d'effluent ; dans lequel le système de commande commande la seconde pompe, la première pompe, ou les deux pompes pour pomper du fluide depuis la source de désinfectant, la source de base et/ou la source d'eau à travers le second module de sorbant ; et dans lequel le système de commande détermine une conductivité de fluide dans la seconde conduite d'effluent sur la base de données provenant du capteur de conductivité positionné dans la seconde conduite d'effluent.

5. Dispositif de recharge de sorbant (1401) selon la revendication 4, dans lequel la seconde conduite d'effluent est reliée fluidiquement à la première conduite d'effluent au niveau d'une jonction ; et comprenant en outre un mélangeur statique au niveau ou en aval de la jonction, de préférence dans lequel le système de commande calcule un rapport de neutralisation sur la base de la conductivité du fluide dans la première conduite d'effluent et de la conductivité du fluide dans la seconde conduite d'effluent ; et dans lequel le système de commande commande la seconde pompe et la première pompe sur la base de données provenant du capteur de conductivité dans la première conduite d'effluent et du capteur de conductivité dans la seconde conduite d'effluent ; dans lequel le système de commande commande la première pompe et la seconde pompe pour générer un fluide doté d'un pH à l'intérieur d'une plage de pH prédéterminée dans le mélangeur statique sur la base du rapport de neutralisation, de préférence dans lequel la plage de pH prédéterminée est comprise entre 5 et 9.

6. Dispositif de recharge de sorbant (1401) selon la revendication 4, dans lequel le système de commande commande la première pompe pour pomper du fluide doté d'un pH acide à travers la première conduite d'entrée et commande la seconde pompe pour pomper du fluide doté d'un pH basique à travers la seconde conduite d'entrée simultanément.

7. Dispositif de recharge de sorbant (1401) selon la revendication 2, dans lequel le système de commande détermine le taux d'écoulement, la pression et la conductivité d'un fluide en amont du premier module de sorbant à des moments prédéfinis.

8. Dispositif de recharge de sorbant (1401) selon la revendication 5, dans lequel le système de commande arrête la seconde pompe lorsque la conductivité du fluide dans la seconde conduite d'effluent atteint une plage prédéterminée, de préférence dans lequel le système de commande arrête la première pompe lorsque la conductivité du fluide dans la première conduite d'effluent atteint une plage prédéterminée, de préférence dans lequel le système de commande démarre la première pompe et la seconde pompe lorsque la conductivité dans la première conduite d'effluent atteint une plage prédéterminée.

9. Dispositif de recharge de sorbant (1401) selon l'une quelconque des revendications précédentes, dans lequel le système de commande commande la première pompe pour pomper de l'eau depuis la source d'eau à travers la première conduite d'entrée après avoir pompé un premier fluide à travers la première conduite d'entrée et avant de pomper un second fluide à travers la première conduite d'entrée.

10. Procédé, comprenant les étapes consistant à :
pomper du fluide depuis une source de désinfectant (1107), une source de base (1108), une source de saumure (1106), une source d'eau (1105) ou des combinaisons de celles-ci, à travers un trajet d'écoulement de recharge (1101) vers un premier module de sorbant (1103) ; et
déterminer la présence d'une fuite, d'une occlusion, d'une défaillance de pompe ou d'un manque de produit chimique, dans lequel la détermination de la présence d'une fuite comprend la détermination du fait qu'une pression dans le trajet d'écoulement de recharge est inférieure à une plage prédéterminée, dans lequel la détermination de la présence d'une occlusion comprend la détermination du fait qu'une pression dans le trajet d'écoulement de recharge (1101) est supérieure à une plage prédéterminée, dans lequel la détermination de la présence d'une défaillance de pompe comprend les étapes consistant à :
déterminer qu'un taux d'écoulement dans le trajet d'écoulement de recharge (1101) est inférieur à une plage prédéterminée ; et
déterminer qu'une conductivité au niveau d'une entrée d'un module de sorbant du premier module de sorbant (1104) se trouve à l'intérieur d'une plage prédéterminée, dans lequel la détermination de la présence d'un manque de produit chimique comprend les étapes consistant à déterminer qu'un taux d'écoulement dans le trajet d'écoulement de recharge (1101) est inférieur à une plage prédéterminée ; et
déterminer qu'une conductivité au niveau d'une entrée de module de sorbant du premier module de sorbant (1103) est inférieure à une plage prédéterminée.

11. Procédé selon la revendication 10, comprenant en outre les étapes de pompage de fluide depuis une source de désinfectant (1107), une source de base (1108), une source de saumure (1106), une source d'eau (1105) ou des combinaisons de celles-ci à travers le trajet d'écoulement de recharge (1101) vers un second module de sorbant (1104) ; et
de pompage de fluide à travers une première conduite d'effluent reliée fluidiquement au premier module de sorbant (1103) et une seconde conduite d'effluent reliée fluidiquement au second module de sorbant (1104) vers un mélangeur statique (1146) ou un réservoir commun.

12. Procédé selon la revendication 11, comprenant en outre :
la détermination d'une conductivité d'un fluide dans la première conduite d'effluent et la détermination et la conductivité d'un fluide dans la seconde conduite d'effluent ; et
le calcul d'un rapport de neutralisation sur la base d'une conductivité de fluide dans la première conduite d'effluent et la seconde conduite d'effluent ;
dans lequel l'étape de pompage de fluide depuis la première conduite d'effluent et la seconde conduite d'effluent vers le mélangeur statique (1146) ou le réservoir commun comprend la commande d'un taux d'écoulement du fluide dans la première conduite d'effluent et la seconde conduite d'effluent sur la base du rapport de neutralisation pour générer un fluide dans le mélangeur statique ou le réservoir commun à l'intérieur d'une plage de pH prédéterminée.

13. Procédé selon l'une quelconque des revendications 10 à 12, comprenant en outre les étapes consistant à :
déterminer une température du fluide, le fluide étant une solution de saumure dans une première conduite d'effluent reliée fluidiquement au premier module de sorbant (1103) ;
calculer une quantité de solution de saumure nécessaire pour recharger le phosphate de zirconium, le premier module de sorbant (1103) contenant du phosphate de zirconium sur la base, au moins en partie, de la température dans la première conduite d'effluent,
de préférence comprenant en outre les étapes consistant à :
pomper du fluide depuis la source de désinfectant (1107) dans le premier module de sorbant (1103) ;
déterminer une conductivité dans une première conduite d'effluent ; et
arrêter l'étape de pompage du fluide depuis la source de désinfectant (1107) si la conductivité dans la première conduite d'effluent se trouve à l'intérieur d'une plage prédéterminée, comprenant éventuellement en outre l'étape de pompage de l'eau depuis la source d'eau (1105) dans le premier module de sorbant (1103) à un moment prédéterminé après l'étape d'arrêt du pompage de fluide depuis la source de désinfectant (1107), comprenant éventuellement en outre l'étape d'aération du premier module de sorbant (1103) à un moment prédéterminé après l'arrêt du pompage de fluide depuis la source de désinfectant (1107), comprenant éventuellement en outre l'étape de pompage de fluide depuis la source de désinfectant (1107) dans le premier module de sorbant (1103) tout en aérant le premier module de sorbant (1103).

14. Procédé selon l'une quelconque des revendications 10 à 13, dans lequel le premier module de sorbant (1103) contient de l'oxyde de zirconium, et le procédé comprend en outre l'étape de détermination d'une quantité de base pompée à travers le premier module de sorbant (1103).
